(19) Europäisches Patentamt
European Patent Office
Office européen des brevets

(11) EP 4 541 286 A1

(12) **EUROPEAN PATENT APPLICATION**

(43) Date of publication:
**23.04.2025 Bulletin 2025/17**

(21) Application number: **24207437.5**

(22) Date of filing: **18.10.2024**

(51) International Patent Classification (IPC):
***A61B 8/00*** *(2006.01)* ***A61B 8/06*** *(2006.01)*
***A61B 8/08*** *(2006.01)*

(52) Cooperative Patent Classification (CPC):
**A61B 8/06; A61B 8/5207; A61B 8/5223**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.10.2023 JP 2023181364**
**20.10.2023 JP 2023181379**
**12.09.2024 JP 2024158402**

(71) Applicant: **CANON KABUSHIKI KAISHA**
**Tokyo 146-8501 (JP)**

(72) Inventors:
- **SATO, Takeshi**
  **Tokyo, 146-8501 (JP)**
- **TAKAHASHI, Hiroki**
  **Tokyo, 146-8501 (JP)**
- **SASAKI, Shoya**
  **Tokyo, 146-8501 (JP)**
- **IIZUKA, Naoya**
  **Tokyo, 146-8501 (JP)**

(74) Representative: **TBK**
**Bavariaring 4-6**
**80336 München (DE)**

(54) **ULTRASONIC DIAGNOSTIC APPARATUS AND IMAGE PROCESSING METHOD**

(57) An ultrasonic diagnostic apparatus according to an embodiment includes a processing circuitry. The processing circuitry reduces the clutter component, which results from the tissues, from a data row of the plurality of frames and estimates first-type blood flow information. Moreover, the processing circuit generates, from the first-type blood flow information, second-type blood flow information representing images of scalar values of blood flow signals, detects the local maximums of the second-type blood flow information, integrates the local maximums and generates a scalar value integration image, calculates an autocorrelation function in the frame direction at positions of the local maximum in the first-type blood flow information, integrates the autocorrelation functions and generates an autocorrelation function integration image, generates third-type blood flow information, in which the blood flow is color-coded, from the scalar value integration image and the autocorrelation function integration image, and displays the third-type blood flow information on a display.

FIG.2

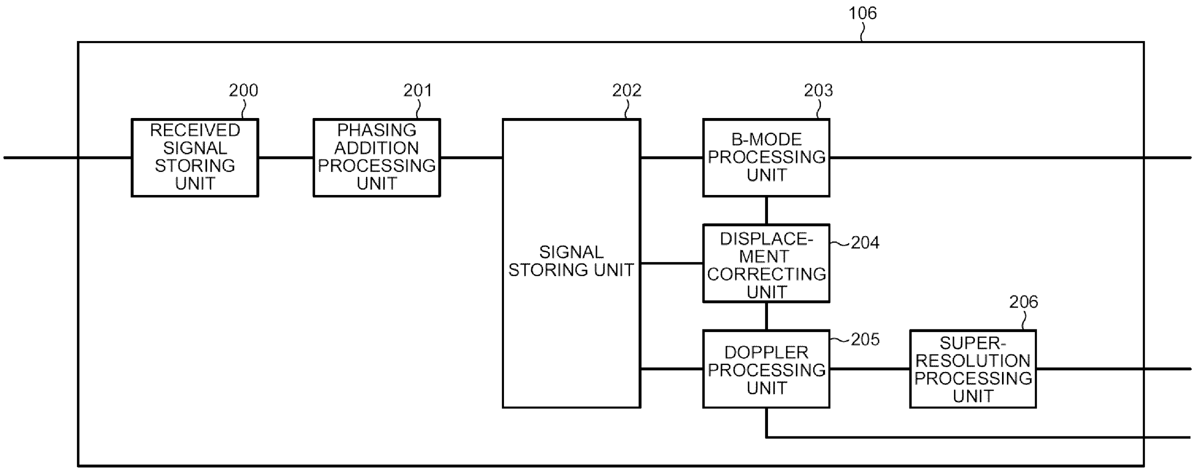


EP 4 541 286 A1

## Description

FIELD

Embodiments described herein relate generally to an ultrasonic diagnostic apparatus and an image processing method.

BACKGROUND

An ultrasonic diagnostic apparatus is widely used for observing or diagnosing the blood flow of a biological body. According to the Doppler method that is based on the Doppler effect, an ultrasonic diagnostic apparatus generates and displays blood flow information from the reflected waves of ultrasonic waves. The blood flow information generated and displayed by an ultrasonic diagnostic apparatus can be in the form of color Doppler images or Doppler waveforms (Doppler spectrums).

A color Doppler image is obtained when imaging is performed according to the color flow mapping (CFM) method. In the CFM method, the transmission of ultrasonic waves is performed over a plurality of scanning lines for a plurality of times. In the CFM method, a moving target indicator (MTI) filter is applied with respect to the data row at the same position; the signals resulting from stationary tissues or from tissues having slow movement (i.e., clutter signals) are suppressed; and the signals resulting from the blood flow (i.e., blood flow signals) are extracted. Then, in the CFM method, from the blood flow signals, blood flow information such as the velocity of the blood flow, the dispersion of the blood flow, and the power of the blood flow is estimated; and the distribution of the estimation result (the blood flow information) is displayed as a Doppler image.

It is a known fact that, in a B-mode image or a Doppler image, the resolution undergoes a decline due to the point spread function (PSF) that is determined according to the wavelength of the transmitted ultrasonic waves or according to the transmission-reception aperture width. Although a solution is available in the form of increasing the frequency of the transmitted ultrasonic waves, there is a limit to the frequency band of the probe. Hence, there are limitations on the achievable resolution in images.

In Non Patent Literature ("Fast Super Resolution Ultrasound Imaging using the Erythrocytes", Jorgen Arendt Jensen, Mikkel Schou, Sofie Bech Andersen, et al., Proceedings Volume 12038, Medical Imaging 2022: Ultrasonic Imaging and Tomography; 120380E (2022)), the explanation is given about a super-resolution technology for blood flow images that enables achieving the resolution of about 1/5-th of the transmitted ultrasonic wavelength. Herein, a large number of normal Doppler images are obtained and the portions having high image values are extracted and integrated, so that a blood flow image having an enhanced resolution is generated. In Non Patent Literature mentioned above, the only thing that is mentioned is performing super-resolution display of the power of the blood flow in grayscale.

BRIEF DESCRIPTION OF THE DRAWINGS

FIG. 1 is a block diagram illustrating an exemplary hardware configuration of an ultrasonic diagnostic apparatus according to a first embodiment;
FIG. 2 is a block diagram illustrating a block diagram illustrating an example of the functions of a received signal processing unit according to the first embodiment;
FIG. 3 is a diagram illustrating an example of the flow of operations performed by the ultrasonic diagnostic apparatus according to the first embodiment for obtaining a super-resolution blood flow image;
FIGS. 4A to 4D are various types of images of the kidney blood flow;
FIG. 5 is a diagram illustrating an example of the flow of operations performed by the ultrasonic diagnostic apparatus according to a second embodiment for obtaining a super-resolution blood flow image;
FIG. 6A is a diagram illustrating an example of blood flow images of a plurality of frames as generated at Step S430 according to the second embodiment;
FIG. 6B is a diagram illustrating an example in which the image groups are divided based on three characteristics according to the second embodiment;
FIG. 6C is a diagram illustrating an example of the images obtained by performing the operation at Step S460 according to the second embodiment;
FIG. 6D is a diagram illustrating an example of a super-resolution blood flow image according to the second embodiment;
FIG. 7 is a diagram illustrating an example of the flow of operations performed to obtain a super-resolution blood flow image according to the conventional technology;

FIGS. 8A to 8C are diagrams for explaining an example of the processing method according to the conventional technology;

FIG. 9 is a diagram illustrating an example of the Reyleigh distribution;

FIGS. 10A to 10C are diagrams for explaining examples of the problems faced in the conventional technology;

FIGS. 11A to 11H are diagrams illustrating images of the kidney blood flow that are obtained according to various methods; and

FIG. 12 is a diagram for explaining the operations performed from Step S450 onward illustrated in FIG. 5.

DETAILED DESCRIPTION

One of the problems to be solved in the embodiments described herein is to obtain information having a high degree of user-friendliness. However, the problem mentioned above is not the only problem that is to be solved in the embodiments described herein. In the embodiments described later, the problems corresponding to the effects achieved due to the disclosed configuration can be treated as the other problems.

Exemplary embodiments and modification examples of an ultrasonic diagnostic apparatus, an image processing device, and an image processing method are described below in detail with reference to the accompanying drawings. However, the ultrasonic diagnostic apparatus, the image processing device, and the image processing method according to the application concerned are not limited by the embodiments and the modification examples described below. The embodiments can be combined with other embodiments, with modification examples, and with the conventional technology without causing any contradictions in the processing details. In an identical manner, the modification examples can be combined with the embodiments, with other modification examples, and with the conventional technology without causing any contradictions in the processing details.

First embodiment

An ultrasonic diagnostic apparatus according to a first embodiment described below includes a processing circuitry. The processing circuitry obtains a data row of reflected-wave data that is taken at one or more positions in a plurality of frames in the time direction by performing ultrasonic transmission and reception. Then, the processing circuit reduces the clutter component, which results from the tissues, from the data row of the plurality of frames and estimates first-type blood flow information. Moreover, the processing circuit generates, from the first-type blood flow information, second-type blood flow information representing images of scalar values of blood flow signals, detects the local maximums of the second-type blood flow information, integrates the local maximums or integrates images in which the local maximums are emphasized and generates a scalar value integration image, calculates an autocorrelation function in the frame direction at positions of the local maximum in the first-type blood flow information, integrates the autocorrelation functions and generates an autocorrelation function integration image, generates third-type blood flow information, in which the blood flow is color-coded, from the scalar value integration image and the autocorrelation function integration image, and displays the third-type blood flow information on a display.

FIG. 1 is a block diagram illustrating an exemplary hardware configuration of the ultrasonic diagnostic apparatus according to the first embodiment. The ultrasonic diagnostic apparatus includes an ultrasonic probe 102, a probe connection 103, a transmission electrical circuit 104, a reception electrical circuit 105, a received signal processing unit 106, an image processing unit 107, a display device 108, and a system control unit 109. The ultrasonic diagnostic apparatus is a system in which ultrasonic pulses are transmitted from the ultrasonic probe 102 onto a subject 100; reflected ultrasonic waves coming from the inside of the subject 100 are received; and image information (ultrasonic images) of the inside of the subject 100 is generated using the reflected ultrasonic waves that are received. The ultrasonic images obtained in an ultrasonic diagnostic apparatus are used in various types of clinical examination.

The ultrasonic probe 102 performs electronic scanning, and includes a plurality of transducers 101 arranged at the front end thereof in one dimension or in two dimensions. The transducers 101 are electromechanical transduction elements that perform reciprocal conversion between electrical signals (voltage pulse signals) and ultrasonic waves (acoustic waves). The ultrasonic probe 102 transmits ultrasonic waves from the transducers 101 onto the subject 100, and receives reflected ultrasonic waves from the subject 100 using the transducers 101. The reflected ultrasonic waves are reflective of the difference in the acoustic impedance inside the subject 100. When the transmitted ultrasonic pulses get reflected from the moving blood flow or from a surface such as the heart wall; due to the Doppler effect, the reflected-wave signals undergo a frequency shift on account of being dependent on the velocity component with respect to the ultrasonic wave transmission direction of the moving body.

The transmission electrical circuit 104 is a transmission unit that outputs pulse signals (driving signals) to the transducers 101. The transmission electrical circuit 104 applies pulse signals onto the transducers 101 at mutually different timings, so that ultrasonic waves having different delay periods are transmitted from the transducers 101. That results in the formation of a transmission ultrasonic beam. The transmission electrical circuit 104 selectively varies the transducer 101 to be applied with pulse signals (i.e., the transducer 101 to be activated) and varies the delay period (the application timing) of the pulse signals, so as to become able to control the direction and the focus of the transmission ultrasonic beam. As a result of sequentially varying the direction and the focus of the transmission ultrasonic beam, the observation region inside the subject 100 gets scanned. Meanwhile, the transmission electrical circuit 104 can vary the delay periods of the pulse signals, and can form a transmission ultrasonic beam that represents a planar wave (having the point of focus at a distant position) or a diffusion wave (having the point of focus on the opposite side of the transducers 101 in the ultrasonic transmission direction). Alternatively, the transmission electrical circuit 104 can form a transmission ultrasonic beam using a single transducer 101 or using some of a plurality of transducers 101. The transmission electrical circuit 104 transmits the pulse signals having a predetermined driving waveform to the transducers 101, and causes generation of transmission ultrasonic waves having a predetermined transmission waveform in the transducers 101. The reception electrical circuit 105 represents a receiving unit that receives input, as received signals, of the electrical signals that are output from the transducers 101 upon receiving the reflected ultrasonic waves. The reception electrical circuit 105 converts the received signals of the analog format (i.e., converts analog signals), which are input from the transducers 101, into received signals of the digital format (i.e., into digital signals); and outputs the received signals of the digital format to the received signal processing unit 106.

In the present written specification, the analog signals output from the transducers 101 as well as the digital data obtained by sampling (digital conversion) of the analog signals are referred to as received signals without particularly distinguishing therebetween. However, depending on the context, when the aim is to clearly specify that digital data is used, the received signals are sometimes referred to as the received data.

The operations of the transmission electrical circuit 104 and the reception electrical circuit 105 are controlled by the system control unit 109. That is, the transmission and reception of the ultrasonic waves is controlled by the system control unit 109. For example, depending on whether a B-mode image (explained later) is to be generated or a blood flow image (explained later) is to be generated, the system control unit 109 varies the voltage signals and varies the position for formation of transmission-type ultrasonic waves.

In the case of generating a B-mode image, the ultrasonic diagnostic apparatus obtains the received signals of the reflected ultrasonic waves obtained by scanning the observation region, and uses those received signals in image generation. In the case of generating a blood flow image, the ultrasonic diagnostic apparatus performs transmission and reception of ultrasonic waves for a plurality of times over each of one or more scanning lines; obtains the received signals of the reflected ultrasonic waves of a plurality of frames; and uses the received signals of the plurality of frames in image generation, that is, in extracting blood flow information. The scanning for generating a blood flow image can be performed either according to a method in which transmission and reception of ultrasonic waves is performed for a plurality of times over a single scanning line and then transmission and reception over the next scanning line is performed in an identical manner; or according to a method in which transmission and reception over each scanning line at a time is repeated for a plurality of times. Alternatively, regarding the generation of B-mode images and blood flow images, in order to reduce the number of scanning lines, for example, planar waves or diffusion waves are transmitted to ensure that ultrasonic waves are transmitted over a wide range of the observation region. Moreover, by varying the transmission angle of the planar waves or the diffusion waves and by varying the range of the observation region to which the transmission is done, the ultrasonic diagnostic apparatus can perform transmission and reception for a plurality of number of times over a wide range of the observation region; and can use the received signals by adding them up.

The received signal processing unit 106 is an image generating unit that generates various types of images (various types of image data) based on the received signals obtained from the ultrasonic probe 102. The image processing unit 107 performs image processing such as luminance adjustment, interpolation, and filter processing with respect to the image data generated by the received signal processing unit 106. The display device 108 is a display unit for displaying the image data and a variety of information, and is configured using one of various types of display such as a liquid crystal display and an organic EL display. The system control unit 109 is a control unit that comprehensively controls the transmission electrical circuit 104, the reception electrical circuit 105, the received signal processing unit 106, the image processing unit 107, and the display device 108.

Configuration of received signal processing block

FIG. 2 is a block diagram illustrating a block diagram illustrating an example of the functions of the received signal processing unit 106 according to the first embodiment. The received signal processing unit 106 includes a received signal storing unit 200, a phasing addition processing unit 201, a signal storing unit 202, a B-mode processing unit 203, a displacement correcting unit 204, a Doppler processing unit 205, and a super-resolution processing unit 206.

The received signal storing unit 200 is used to store the received signals output from the reception electrical circuit 105. Meanwhile, depending on the device configuration and the type of received signals, instead of storing the received signals in the received signal storing unit 200, the received signals that have been processed by the phasing addition processing unit 201 (explained later) can be stored in the signal storing unit 202. Moreover, the received signal storing unit 200 can be configured from the same block as the block for the signal storing unit 202, so that the received signals coming from the reception electrical circuit 105 as well as the received signals that have been processed by the phasing addition processing unit 201 can be stored in the same block.

The phasing addition processing unit 201 performs phasing addition and quadrature detection with respect to the received signals that are obtained by the reception electrical circuit 105, and stores the post-processing received signals in the signal storing unit 202. In the phasing addition, the delay period and the weight for each transducer 101 is varied and the received signals of a plurality of transducers 101 are added, so that a reception ultrasonic beam is formed. The phasing addition is also called delay and sum (DAS) beam formation. In the quadrature detection, the received signals are converted into in-phase signals (I signals) and quadrature signals (Q signals). The phasing addition and the quadrature detection are performed based on various conditions (such as aperture control and signal filtering) for element arrangement and image generation as input from the system control unit 109. The received signals that have been subjected to phasing addition and quadrature detection are stored in the signal storing unit 202. Herein, DAS beam formation is explained as the representative example. However, as long as a reception ultrasonic beam can be formed, the phasing addition processing unit 201 can perform any other operation such as adaptive beam formation, model-based processing, or processing using machine learning.

The B-mode processing unit 203 performs B-mode processing such as envelope detection and logarithmic compression with respect to the received signals that are stored in the signal storing unit 202 and that are to be used in generating B-mode images; and generates image data in which the signal intensity at each point in the observation region is expressed in the form of luminance. The B-mode processing unit 203 can perform B-mode processing also with respect to the received signals that have been corrected for displacement by the displacement correcting unit 204 (explained below).

The displacement correcting unit 204 calculates, from the received signals of a plurality of frames, the amount of displacement of the tissues occurring across the frames due to the body motion. The amount of displacement of the tissues is calculated according to, for example, block matching computation called the spectral tracking method. The displacement correcting unit 204 sets a region of interest in each frame, tracks the correlation among the regions of interest in the frames, and calculates the displacement occurring in the regions of interest. Moreover, the displacement correcting unit 204 sets a plurality of regions of interest in a frame, and calculates the displacement of the entire frame. Herein, the explanation is given about an exemplary method in which the correlation among the frames is used. However, as long as the displacement of the regions of interest can be obtained, any other method can also be implemented. Regarding ultrasonic signals, the spectrum that represents a scattering image of the ultrasonic waves reflected from the scattering substance in the body is tracked. Hence, the tracking is called spectral tracking. Meanwhile, the correlation computation of the regions of interest among the frames can also be performed with respect to the received signals that have been subjected to phasing addition, or quadrature detection, or envelope detection. Moreover, the displacement correcting unit 204 can perform correlation computation with respect to the time waveform of the received signals, or can perform correlation computation with respect to the frequency space data obtained by performing discrete Fourier transformation with respect to the received signals. Then, using the calculated displacement, the displacement correcting unit 204 moves the received signals with respect to the reference frame and corrects the displacement. The amount of displacement to be corrected either can be kept uniform over the entire frame, such as the average displacement of all regions of interest; or can be varied for each region of interest in the frame. Moreover, the displacement of the entire frame as calculated for each region of interest can be interpolated within the frame as well as in the time direction of the data row of a plurality of frames using linear interpolation or spline interpolation. Furthermore, at the time of correcting the displacement too, the displacement correcting unit 204 can interpolate the received signals in an identical manner so as to enable more minute correction, and then move the received signals with respect to the reference frame.

The doppler processing unit 205 extracts blood flow information (Doppler information) from the received signals that are stored in the signal storing unit 202 and that are to be used in generating blood flow images; and generates a blood flow image (blood flow image data) in which the blood flow information is captured. Moreover, the Doppler processing unit 205 can perform Doppler processing with respect to the received signals that have been corrected for displacement by the displacement correcting unit 204.

Given below is the detailed explanation about the operation details of the Doppler processing unit 205. The Doppler processing unit 205 performs frequency analysis of the received signals that are stored in the signal storing unit 202 and that are to be used in generating Doppler images; and extracts blood flow information based on the Doppler effect of the object present within the scanning range. In the first embodiment, the explanation is mainly given about the example in which the blood represents the object. However, the body tissues or the contrast dye can also be treated as the object. Moreover, examples of the blood flow information include at least either the velocity, or the dispersion value, or the power value. Meanwhile, the Doppler processing unit 205 either can obtain the blood flow information at a single point (at a single position) inside the subject, or can obtain the blood flow information at a plurality of positions in the depth direction. Moreover, the Doppler processing unit 205 can obtain the blood flow information at a plurality of timings in a chronological order, so that the time variation in the blood flow information can be displayed.

In the generation of a blood flow image according to the Doppler method, at one or more positions, the received-signal data row of a plurality of frames is obtained in the time direction. The Doppler processing unit 205 applies a moving target indicator (MTI) filter with respect to the received-signal data row; reduces the tissues that have become stationary among the frames or reduces the component resulting from the tissues having less movement (i.e., the clutter component); and extracts the component resulting from the blood flow. Then, from that blood flow component, the Doppler processing unit 205 calculates the blood flow information such as the velocity of the blood flow, the dispersion of the blood flow, and the power of the blood flow.

Regarding the MTI filter, it is possible to use a filter having a fixed filter coefficient such as an infinite impulse response (IIR) filter of the Butterworth type or a polynomial regression filter; or it is possible to use an adaptive filter that varies the coefficient according to the input signals using eigenvalue decomposition or singular value decomposition; or it is possible to decompose the received signal data into one or more bases using eigenvalue decomposition or singular value decomposition, and to retrieve only a particular base and remove the clutter component.

Moreover, by implementing a method such as the vector Doppler method, the spectral tracking method, or the vector flow mapping method, the Doppler processing unit 205 can obtain the velocity vector at each coordinate in an image and obtain a blood flow vector indicating the magnitude and the orientation of the blood flow.

From the blood flow image data generated by the Doppler processing unit 205, the super-resolution processing unit 206 generates super-resolution blood flow image data representing a blood flow image having enhanced resolution. Regarding the method of obtaining a super-resolution blood flow image according to the first embodiment, the explanation is given later at the time of explaining the flow of operations.

The image data output from the B-mode processing unit 203, the Doppler processing unit 205, and the super-resolution processing unit 206 is processed by the image processing unit 107; and then all processed image data is eventually displayed on the display device 108. Herein, the sets of image data either can be displayed in a superimposed manner or can be displayed side by side, or only some sets of image data can be displayed.

The received signal processing unit 106 can be configured with one or more processors and a memory. In that case, the function of the phasing addition processing unit 201 and the functions of the B-mode processing unit 203 to the super-resolution processing unit 206 are implemented using a computer program. Moreover, the received signal storing unit 200 and the signal storing unit 202 illustrated in FIG. 2 are implemented using a memory. For example, a CPU can read a computer program stored in a memory and execute the computer program so that the function of the phasing addition processing unit 201 and the functions of the B-mode processing unit 203 to the super-resolution processing unit 206 can be provided. Meanwhile, in addition to including a CPU, the received signal processing unit 106 can also include a processor (such as a GPU or an FPGA) for handling the calculations of the B-mode processing unit 203, the displacement correcting unit 204, the Doppler processing unit 205, and the super-resolution processing unit 206. The memory can include a memory for storing computer programs in a non-permanent manner, a memory for temporarily storing data such as the received signals, and a working memory used by the CPU.

Till now, the explanation was given about the overall configuration of the ultrasonic diagnostic apparatus according to the first embodiment. Given below is the explanation of the flow of operations performed to obtain a super-resolution blood flow image according to the first embodiment.

Flow of operations performed to obtain a super-resolution blood flow image according to the first embodiment

Explained below with reference to FIG. 3 is the flow of operations performed to obtain a super-resolution blood flow image according to the first embodiment. FIG. 3 is a diagram illustrating an example of the flow of operations performed by the ultrasonic diagnostic apparatus according to the first embodiment for obtaining a super-resolution blood flow image.

The ultrasonic probe 102 performs transmission and reception of ultrasonic waves at the same position in a repeated manner so as to ensure that the target region for obtaining the blood flow information is included; and the reception electrical circuit 105 and the phasing addition processing unit 201 create received signals that include the data row of a plurality of frames in the time direction (Step S310). That is, the reception electrical circuit 105 and the phasing addition processing unit 201 obtains the data row of the reflected-wave data taken at one or more positions in a plurality of frames in the time direction by performing ultrasonic transmission and reception. For example, the reception electrical circuit 105 and the phasing addition processing unit 201 represent an example of an obtaining unit. In the first embodiment, at the time of generating a super-resolution blood flow image, the frame count in the data row sometimes becomes greater than the frame count at the time of generating a normal Doppler image. For example, at the time of generating a normal Doppler image, the frame count in the data row is in the range of about five to 20. In contrast, at the time of generating a super-resolution blood flow image, there are times when a few hundred frames to a few tens of thousands of frames of the data row are required. The received signals including the data row of a plurality of frames are subjected to phasing addition and quadrature detection by the phasing addition processing unit 201; and the received signals that have been subjected to phasing addition and quadrature detection are stored in the signal storing unit 202. Regarding a single frame in the data row, either the received signals obtained when ultrasonic transmission/reception is performed once to ensure that the observation region is included can be treated as a single frame, or the result of addition of the received signals that have been transmitted and received for a plurality of times for including the observation region can be treated as a single frame. For example, the ultrasonic probe 102 transmits planar waves or diffusion waves so as to ensure that the observation region is included; and the phasing addition processing unit 201 adds a plurality of received signals having varied transmission angles and treats the added signals as the data of a single frame.

From the received signals including the data row of a plurality of frames, the displacement correcting unit 204 calculates the displacement occurring in the tissues across the frames due to the body motion; and, using the calculated displacement, corrects the displacement by moving the received signals in such a way that the position of the tissues is same across the frames which are to be subjected to integration at Step S370 (explained later) (Step S320). At that time, a single frame in the entire data row can be treated as the reference frame for calculating the displacement, or a plurality of reference frames can be set by varying them according to the position in the time direction, or the reference frame can be calculated between adjacent frames.

The Doppler processing unit 205 reduces the clutter component by applying an MTI filter with respect to the received signals that include the data row of a plurality of frames which have been corrected for displacement by the displacement correcting unit 204; and extracts the components resulting from the blood flow and generates blood flow images of a plurality of frames (Step S330). At that time, in the design of the MTI filter, either some part of the data row can be retrieved and used, or the entire data row can be used. In this way, at Step S330, the Doppler processing unit 205 reduces the clutter component, which results from the tissues, from the data row of a plurality of frames, and estimates first-type blood flow information.

The Doppler processing unit 205 increases the reception scanning lines by means of interpolation and thus enhances the resolution of the local maximums (explained later) (Step S340). Since the blood flow signals are complex signals, the interpolation in the scanning direction results in phased interpolation, and the resolution is enhanced. Under normal conditions, it is better to increase the number of reception scanning lines during beam formation. However, performing interpolation at this point of time enables achieving reduction in the processing load. That is, interpolation is performed not at the timing of performing beam formation but at a timing after performing beam formation, so that the processing load at the time of beam formation can be reduced. In the case in which a sufficient number of reception scanning lines are secured for beam formation, the operation at Step S340 need not be performed.

Subsequently, the super-resolution processing unit 206 extracts (detects) the local maximums of the power values of each frame and, at the same time, calculates autocorrelation functions of the positions of the local maximums (Step S350). Herein, the local maximum implies, for example, the maximum power value from among the power values in each of a plurality of local regions of a frame. Then, the super-resolution processing unit 206 integrates, on a frame-by-frame basis, the local maximums of the power values and the value of the autocorrelation function (Step S360). A post-addition power value c0(x, y) at a position (x, y) and a post-addition autocorrelation function c1(x, y) can be expressed as Equation (1) and Equation (2) given below.

$$\mathrm{c0}(\mathrm{x},\mathrm{y}) = \sum_{n=1}^{N} z(x,y,n) z^{*}(\mathrm{x},\mathrm{y},\mathrm{n}) \mathrm{p}(\mathrm{x},\mathrm{y},\mathrm{n}) \quad \cdots (1)$$

$$\mathrm{c1}(\mathrm{x},\mathrm{y}) = \sum_{n=2}^{N} z(x,y,n) z^{*}(\mathrm{x},\mathrm{y},\mathrm{n}-1) \mathrm{p}(\mathrm{x},\mathrm{y},\mathrm{n}) \quad \cdots (2)$$

Herein, z(x, y, n) represents the IQ signal (a complex signal of the baseband, where "I" stands for In-phase and "Q" stands for Quadrature phase) of the input, which is received at Step S350, at the position (x, y) of the n-th frame. Moreover, "*" represents the complex conjugate. Furthermore, p(x, y, n) represents a function that returns "1" when the point at the position (x, y) of the n-th frame is the local maximum, and returns "0" if that point is not the local maximum. Moreover, N represents the total number of frames to be processed.

In this way, at Steps S350 and S360, from the first-type blood flow information, the super-resolution processing unit 206 generates second-type blood flow information that represents an image of the power values which are the scalar values of the blood flow signals. Alternatively, from the first-type blood flow information, the super-resolution processing unit 206 can generate, as the second-type blood flow information, an image of the amplitudes of the blood flow signals representing the scalar values of the blood flow signals. Still alternatively, from the first-type blood flow information, the super-resolution processing unit 206 can generate, as the second-type blood flow information, images of other scalar values of the blood flow signals. Meanwhile, at Step S360, instead of integrating the images in which the local maximums are extracted, the super-resolution processing unit 206 can integrate the images in which the local maximums are emphasized by attenuating the pixel values other than the local maximums. The resultant image is used in the operation performed at Step S370 (explained later). Meanwhile, although the original images also get included to a certain extent, the attenuation amount can be set to infinity and only the pixel values of the local maximum points can be extracted. At Step S360, the super-resolution processing unit 206 detects the image points within a predetermined range or detects the areas of the local maximum points from the second-type blood flow information, and creates an image in which the pixel values of the local maximum points are emphasized. The image points within a predetermined range can represent the portion having the maximum image value in each image, or can represent the positions of the image values within such a range for which a threshold value is set with respect to the maximum image value. Meanwhile, if the pixel value of the point of interest is greater than the adjacent pixel values, then the point of interest can be treated as the local maximum point. Moreover, the maximum point in each local region can be extracted, so that a plurality of local maximum points is extracted within the image.

At Steps S350 and S360, the super-resolution processing unit 206 detects the local maximums of the second-type blood flow information, and integrates the local maximums to generate a scalar value integration image. Moreover, at Steps S350 and S360, at positions corresponding to the local maximum, the super-resolution processing unit 206 calculates the autocorrelation function in the frame direction (the time axis direction) of the first-type blood flow information, and integrates the autocorrelation functions to generate an autocorrelation function integration image. Furthermore, the super-resolution processing unit 206 detects the local maximums of the power of the second-type blood flow information, and integrates the local maximums to generate a power value integration image as a scalar value integration image.

Then, the super-resolution processing unit 206 uses the integrated power value and the integrated autocorrelation function value, and performs color coding of the power values (Step S370). For example, the super-resolution processing unit 206 calculates the blood flow velocities from the autocorrelation function values, performs RGB conversion according to a two-dimensional colormap of the blood flow velocities (the blood flow velocity values) and the power values, and displays an image. That is, the super-resolution processing unit 206 displays, on the display device 108, the image obtained by performing RGB conversion according to a two-dimensional colormap. The power values for display are calculated by performing logarithmic compression of c0(x, y) given in Equation (1). The velocity values for display are calculated from the argument of the complex number of c1(x, y) given in Equation (2).

At Step S370, the super-resolution processing unit 206 calculates only the direction of the blood flow from the autocorrelation function values, performs color coding according to the direction, and displays the power values on the display device 108. The direction of the blood flow is determined by the sign of the imaginary part of the autocorrelation function. Hence, when only the direction of the blood flow is required, the super-resolution processing unit 206 can obtain the imaginary part $cl_{im}(x, y)$ of the autocorrelation function cl(x, y) according to Equation (3) given below, and can calculate only the direction of the blood flow according to the sign of the imaginary part $cl_{im}(x, y)$.

$$c1_{im}(x,y) = \sum_{n=2}^{N} \{z_{im}(x,y,n) z_{re}(x,y,n-1) - z_{re}(x,y,n) z_{im}(x,y,n-1)\} p(x,y,n) \quad \cdots (3)$$

Herein, the subscripted "re" represents the real part, and the subscripted "im" represents the imaginary part.

As explained above, at Step S370, from the scalar value integration image and the autocorrelation function integration image, the super-resolution processing unit 206 generates third-type blood flow information which has higher resolution than the resolution of the first-type blood flow information and in which the blood flow is color-coded; and displays the third-type blood flow information on the display device 108. Moreover, at Step S370, the super-resolution processing unit 206 calculates the information about the direction of the blood flow (the information related to the direction of the blood flow) from the autocorrelation function integration image; and, from the scalar value integration image and the information about the direction of the blood flow, generates third-type blood flow information in which color coding is done according to the direction of the blood flow. That is, from the scalar value integration image and the information related to the direction of the blood flow at positions corresponding to the local maximum, the super-resolution processing unit 206 generates third-type blood flow information in which color coding is done according to the direction of the blood flow.

Explained below with reference to FIGS. 4A to 4D are the effects achieved by the ultrasonic diagnostic apparatus according to the first embodiment. FIGS. 4A to 4D are various types of images of the kidney blood flow. The image illustrated in FIG. 4A is an exemplary image for grayscale display (i.e., a grayscale image) obtained according to the conventional technology in which super-resolution is not achieved. The image illustrated in FIG. 4B is an exemplary image for color display with direction (i.e., a color image) according to the conventional technology in which super-resolution is not achieved. The image illustrated in FIG. 4C represents an exemplary image for grayscale display without direction but with super-resolution. The image illustrated in FIG. 4D represents an exemplary image for super-resolution display according to the first embodiment in which color coding is done depending on the direction.

In the image illustrated in FIG. 4D, the region hatched using positively sloped lines is displayed in, for example, red color and represents the region of the renal arteries flowing in the direction toward the ultrasonic probe 102. Moreover, the region hatched using negatively sloped lines is displayed in, for example, blue color and represents the region of the renal veins flowing in the direction away from the ultrasonic probe 102. As compared to the image illustrated in FIG. 4A or the image illustrated in FIG. 4B, the image illustrated in FIG. 4D has significantly higher resolution. Moreover, as compared to the image illustrated in FIG. 4C, color coding is done according to the direction. Hence, the renal arteries flowing in the direction toward the ultrasonic probe 102 and the renal veins flowing in the direction away from the ultrasonic probe 102 can be expressed in a distinguishing manner, thereby resulting in an increase in the volume of information. In this way, as compared to the case in which only the power values of the blood flow are displayed in grayscale and with super-resolution, when the power values of the blood flow are displayed in a color-coded manner according to the velocity and the direction of the blood flow, if the blood vessels running side by side include arteries and veins, the blood vessels can be separated by different colors and can be displayed in an easy-to-understand manner.

The explanation given above is about the case in which, from among the blood flow information, the ultrasonic diagnostic apparatus makes use of only the directions of the blood flow. Alternatively, the Doppler processing unit 205 can calculate blood flow velocity v(x, y) according to Equation (4) given below; and the ultrasonic diagnostic apparatus can perform the display using a colormap of only the velocities or using a two-dimensional colormap of the velocities and the powers.

$$\mathrm{v(x,y) = angle(c1(x,y))} \quad \cdots (4)$$

Herein, angle(c1) represents the function for calculating the argument of the complex number c2.

Moreover, the super-resolution processing unit 206 can calculate dispersion σ(x, y) according to Equation (5) given below; and the ultrasonic diagnostic apparatus can perform the display using a two-dimensional colormap of the velocities and the dispersions.

$$\sigma(\mathrm{x,y}) = 1 - \frac{|c1(x,y)|}{c0(x,y)} \quad \cdots (5)$$

In this way, the super-resolution processing unit 206 calculates the velocities and the dispersions of the blood flow from the power value integration image and the autocorrelation function integration image. Meanwhile, the super-resolution processing unit 206 can calculate the information about the directions of the blood flow from the autocorrelation function integration image, and can perform color display of two or three items from among the velocities, the dispersions, and the power values as the third-type blood flow information on the display device 108.

Till now, the description about the first embodiment was given. According to the first embodiment, the power of the blood flow can be color-coded according to the direction of the blood flow or the velocity of the blood flow, and can be displayed with super-resolution. That is, according to the first embodiment, the blood flow can be color coded and can be displayed with super-resolution. Hence, according to the first embodiment, it becomes possible to obtain information having a high degree of user-friendliness.

Second embodiment

Given below is the description of a second embodiment. Generally, in a blood flow image based on the Doppler method, thick blood vessels having high flow velocity are known to have a higher image value (pixel value) as compared to thin blood vessels having low flow velocity. Hence, in the technology disclosed in Non Patent Literature mentioned earlier, when blood vessels having a difference in the flow velocity, that is, having a difference in the diameter are included adjacent to each other, only the thick blood vessels having high flow velocity are extracted, and the thin blood vessels having low flow velocity do not appear in a blood flow image having enhanced resolution. Moreover, in Non Patent Literature, there is no explanation about the method for using the difference in the directions of the blood flow and the difference in the velocities of the blood flow in achieving super-resolution.

In that regard, as an ultrasonic diagnostic apparatus, an image processing device, and an image processing method according to the second embodiment; the explanation is given about an ultrasonic diagnostic apparatus, an image processing device, and an image processing method that enable displaying blood flow information (blood flow images) with enhanced resolution regarding blood vessels having the flow velocities over a wide range or blood vessels having various diameters or shapes. In the description of the second embodiment, the identical configuration to the first embodiment is not explained again, and the explanation is given mainly about the difference in the configuration from the first embodiment.

In the second embodiment, image groups are divided based on the characteristics having the flow velocity range of each of a plurality of blood flow images as the base; the operation of emphasizing a predetermined numerical range or emphasizing the image values corresponding to the local maximums is performed in each image of the image groups based on the characteristics; and the emphasized image value corresponding to each base are added up. In the second embodiment, since the image value is extracted from each image of the image group that is based on the characteristics divided according to the flow velocity range, the blood vessels having high flow velocity as well as the blood vessels having low flow velocity get extracted, thereby enabling achieving enhancement in the resolution.

As explained below, the ultrasonic diagnostic apparatus according to the second embodiment includes processing circuitry. The processing circuitry obtains reflected-wave data taken at one or more positions in a plurality of frames in the time direction by performing ultrasonic transmission and reception. Then, from the data row of a plurality of frames, the processing circuitry reduces the clutter component resulting from the tissues and estimates the first-type blood flow information. Moreover, the processing circuitry performs orthogonal transformation in the frame direction and calculates the second-type blood flow information from the first-type blood flow information; creates a scalar value image for each frequency of the second-type blood flow information; detects the local maximum point in the image of scalar values at each frequency and performs an operation to emphasize the local maximum points; generates third-type blood flow information from frequency information and from the image in which the local maximum point of the scalar values at each frequency is emphasized; and displays the third-type blood flow information on the display.

In the second embodiment, from the blood flow image data generated by the Doppler processing unit 205, the super-resolution processing unit 206 generates super-resolution blood flow image data representing a blood flow image having enhanced resolution. Regarding the method for obtaining a super-resolution blood flow image according to the conventional technology and according to the second embodiment, the explanation is given below in the explanation of the flow of operations. Given below is the explanation of the flow of operations performed for obtaining a super-resolution blood flow image according to the second embodiment.

Flow of operations performed to obtain a super-resolution blood flow image according to the second embodiment

Explained below with reference to FIG. 5 is the flow of operations performed to obtain a super-resolution blood flow image according to the second embodiment. FIG. 5 is a diagram illustrating an example of the flow of operations performed by the ultrasonic diagnostic apparatus according to the second embodiment for obtaining a super-resolution blood flow image. Meanwhile, the operations at Steps S420 and S440 that are enclosed in dotted blocks can be omitted.

The ultrasonic diagnostic apparatus according to the second embodiment performs the operation at Step S410. The operation performed at Step S410 is identical to the operation performed at Step S310 explained earlier.

From the received signals that include the data row of a plurality of frames, the displacement correcting unit 204 calculates the displacement of the tissues occurring across the frames due to the body motion; and corrects the displacement by moving the received signals (Step S420). At that time, a single frame over the entire data row can be treated as the reference frame for calculating the displacement, or a plurality of reference frames can be set by varying them according to the position in the time direction, or the reference frame can be calculated between adjacent frames.

The Doppler processing unit 205 reduces the clutter component by applying an MTI filter with respect to the received signals that include the data row of a plurality of frames which have been corrected for displacement by the displacement correcting unit 204; and extracts the components resulting from the blood flow and generates blood flow images of a plurality of frames (Step S430). At that time, in the design of the MTI filter, either some part of the data row can be retrieved and used, or the entire data row can be used. In this way, at Step S430, the Doppler processing unit 205 reduces the clutter component, which results from the tissues, from the data row of a plurality of frames, and estimates the first-type blood flow information. Regarding blood flow images of a plurality of frames as generated at Step S430, an example is illustrated in FIG. 6A. Thus, FIG. 6A is a diagram illustrating an example of blood flow images of a plurality of frames as generated at Step S430 according to the second embodiment.

In FIG. 6A, at a position 401 at which the blood flow information is included, the color shade at positions in the image is attributed to the difference in the flow velocity. Hence, at a position having a large image value (having a dark color shade), the flow velocity is high; and, at a position having a small image value (having a light color shade), the flow velocity is low.

Herein, for the sake of comparison, firstly the explanation is given about the flow of operations performed to obtain a super-resolution blood flow image according to the conventional technology and about the problems faced in those operations. Then, the explanation about the flow of operations performed to obtain a super-resolution blood flow image according to the second embodiment is continued. FIG. 7 is a diagram illustrating an example of the flow of operations performed to obtain a super-resolution blood flow image according to the conventional technology. FIGS. 8A to 8C are diagrams for explaining an example of the processing method according to the conventional technology. In FIG. 7, the operations performed at Step S510 to S530 are identical to the operations performed at Steps S410 to S430, respectively. In FIG. 8A are illustrated the blood flow images of a plurality of frames as obtained by performing the operations at Steps S510 to S530 illustrated in FIG. 7. Thus, the blood flow images of a plurality of frames as illustrated in FIG. 8A are identical to the blood flow images of a plurality of frames as illustrated in FIG. 6A.

In each blood flow image of a plurality of frames, the positions having the image values within a predetermined range are extracted (Step S540). The image values within a predetermined range can represent the portion having the maximum image value in each image, or can represent the positions of the image values within such a range for which a threshold value is set with respect to the maximum image value. That is, in each blood flow image, the area having the image values equal to or greater than a certain value is extracted, so that the images as illustrated in FIG. 8B are obtained. In FIG. 8B, the portion illustrated in dashed lines represents the position 401 at which the blood flow information is included as illustrated in FIG. 8A.

Since the area having the image values within a predetermined range moves across the frames, a plurality of blood flow images from which the image values are extracted are integrated so that, as compared to the blood flow images, that is, normal Doppler images, a super-resolution blood flow image having enhanced resolution is generated and displayed as illustrated in FIG. 8C (Step S550). Herein, since only the portion having high image values is extracted, if the point spread function is assumed to have the Gaussian distribution, it means that only the area present in the center of the distribution and having high sharpness is extracted. In a single-frame image, only a point or a small range gets extracted. However, since the portion having high images moves across the frames, when a plurality of blood flow images from which the images values are extracted is integrated, a super-resolution blood flow image is obtained.

As illustrated in FIGS. 8B and 8C, the problem in the conventional technology is that, when the area having the image values equal to or greater than a certain value is extracted from each blood flow image, the blood vessels only having large image values, that is, the blood vessels only having high flow velocity are extracted.

The problem in the conventional technology is explained in detail. FIG. 9 is a diagram illustrating an example of the Reyleigh distribution. Generally, the amplitude distribution characteristics of ultrasonic received signals coming from a homogeneous scattering medium is known to be nearly approximatable with a probability distribution called the Reyleigh distribution illustrated in FIG. 9. At Step S540, extraction of the area having large amplitude values as the image values within a predetermined range implies extraction, from among reflected signals resulting from a large number of red blood cells, of the area having low probability in the Reyleigh distribution but having large amplitude values, so that reflected areas resulting only from a small number of red blood cells are extracted and are spatially sparsed. However, when the blood vessels having a difference in the flow velocity are present adjacent to each other, if the area having large amplitude values is extracted as the area having the image values within a predetermined range, only such reflected signals of thick blood vessels having high flow velocity are extracted which are in the area that has low probability but high amplitude value in the Reyleigh distribution. Hence, the reflected signals of thin blood vessels having low flow velocity do not get extracted.

In FIG. 10A is illustrated an example of a blood flow image in the case in which a thick blood vessel having high flow velocity and a thin blood vessel having low flow velocity are running side by side in the depth direction. The thin blood vessel having low flow velocity is placed at a shallower position, and the thick blood vessel having high flow velocity is placed at a deeper position. However, since the gap between the two blood vessels is equal to or smaller than the resolution, they are inseparable in the blood flow image. FIG. 10B is a diagram illustrating an example of a super-resolution blood flow image that is generated by performing the operations at Steps S540 and S550 with respect to the blood flow images of a plurality of frames according to the conventional technology. As compared to a super-resolution blood flow image generated according to the second embodiment (explained later) and illustrated in FIG. 10C, in the super-resolution blood flow image generated according to the conventional technology, it can be seen that the resolution of only the thick blood vessel having high flow velocity is enhanced, and the thin blood vessel having low flow velocity is not captured.

In order to ensure that the blood vessels having small image values, that is, the blood vessels having low flow velocity are also extracted, it is possible to think of expanding the predetermined range of image values. However, in regard to the extraction with respect to the Gaussian distribution mentioned earlier, since the scope of the extracted distribution expands, the effect of enhancement in the resolution diminishes.

In view of the problems explained above, regarding the flow of operations performed to obtain a super-resolution blood vessel image according to the second embodiment, the explanation is continued below. The Doppler processing unit 205 performs the operation at Step S440. The operation performed at Step S440 is identical to the operation performed at Step S340 explained earlier. Meanwhile, in an identical manner to the first embodiment, in the case in which a sufficient number of reception scanning lines are secured for beam formation, the operation at Step S440 need not be performed.

The super-resolution processing unit 206 divides the blood flow images of a plurality of frames into images having different flow velocity ranges (Step S450). More particularly, the super-resolution processing unit 206 uses some of the frames from among a plurality of frames of the blood flow images, and performs discrete Fourier transformation with respect to the variation of the image value of each pixel in the time direction. The discrete Fourier transformation has the sinusoidal wave base, and expresses the frequency components. For that reason, for each frequency component, that is, for each flow velocity component of the inter-frame variation of each pixel of the blood flow images, the super-resolution processing unit 206 can separate the images. That is, at Step S450, the super-resolution processing unit 206 can separate the images holding the blood flow information of the flow velocity range corresponding to each base. The images holding the blood flow information of the flow velocity range corresponding to each base can be the images of the frequency region after performing discrete Fourier transformation, or can be the images of the time region obtained by performing reverse discrete Fourier transformation with respect to the images of the frequency region. Herein, the explanation is given about the method in which reverse discrete Fourier transformation is not performed and processing is performed using the images of the frequency region. From among the blood flow images of a plurality of frames, some frames to be used in discrete Fourier transformation are varied in the time direction, so that chronologically different images corresponding to the bases are obtained. Herein, those images are called the image groups based on the characteristics. In the second embodiment, the flow velocities are treated as the characteristics, that is, as the bases. In FIG. 6B is illustrated an example in which the image groups are divided based on the three characteristics. In FIG. 6B, in each base, the blood flow having a different flow velocity is captured. The super-resolution processing unit 206 increases the division count, that is, increases the number of frames used in discrete Fourier transformation, so that the flow velocity range corresponding to each base becomes smaller thereby enabling division of blood vessels in smaller flow velocity ranges. Meanwhile, if the base enables division according to the flow velocity range, then it is possible to implement some other method other than discrete Fourier transformation having the sinusoidal wave as the base. Thus, using polynomial regression such as the Legendre polynomial, the blood flow images can be divided into image groups based on the characteristics of the flow velocity ranges. As explained earlier, at Step S450, the super-resolution processing unit 206 performs Fourier transformation in the frame direction with respect to the first-type blood flow information and calculates the second-type blood flow information. Meanwhile, instead of performing Fourier transformation, the super-resolution processing unit 206 can perform some other orthogonal transformation. Moreover, for each frequency in the second-type blood flow information, the super-resolution processing unit 206 creases a scalar value image. The frequency-by-frequency images often represent complex numbers. However, the images to be used next at Step S460 are converted into images in which scalar values such as the power values (the sum of the squares of the real part and the imaginary part) are treated as the pixels in order to enable magnitude comparison. According to the Parseval's theorem, the power values have the property of having the identical integral on the time axis and on the frequency axis, thereby making the power values easy to handle. Hence, the following explanation is given with reference to the power values.

With respect to each image of the power image group based on the characteristics of each base, the super-resolution processing unit 206 detects the image points within a predetermined range or detects the area of the local maximum point and creates an image having the pixel value of the local maximum point emphasized (i.e., creates a peak emphasizing image) (Step S460). The image points within a predetermined range can represent the portion having the maximum image value in each image, or can represent the positions of the image values within such a range for which a threshold value is set with respect to the maximum image value. Meanwhile, if the pixel value of the point of interest is greater than the adjacent pixel values, then the point of interest can be treated as the local maximum point. Moreover, the maximum point in each local region can be extracted, and a plurality of local maximum points can be extracted within the image. Subsequently, an image in which the local maximum points are emphasized (i.e., a peak emphasizing image) is created. Alternatively, for example, a peak emphasizing image can be created by attenuating, by a certain amount, the pixel values other than the local maximum points. As a result, although the original image also gets included to a certain extent, the attenuation amount can be set to infinity and only the pixel values of the local maximum points can be extracted.

In FIG. 5, as the operations subsequent to the operation performed at Step S460, three operations are illustrated. At Steps S470 and S480, the super-resolution processing unit 206 adds up the images in which the local maximum points of all frequencies are emphasized, and displays the resultant image on the display device 108. Regarding the operations performed at Steps S470 and S480, the detailed explanation is given later.

At Steps S490 and S491, the super-resolution processing unit 206 adds up the local maximums separately for positive frequencies and negative frequencies, and performs display on the display device 108 with different color coding for positive frequencies and negative frequencies. That is, at Steps S490 and S491, for each type of frequency, the super-resolution processing unit 206 adds up the peak emphasizing images; performs different color coding of the resultant images according to positive frequencies and negative frequencies; and displays, on the display device 108, a color-coded image of positive frequencies and a color-coded image of negative frequencies. At Steps S492 and S493, the super-resolution processing unit 206 divides the frequencies into a plurality of groups, adds the local maximum values in each group, performs different color coding for each group, and displays the images on the display device 108. That is, at Steps S492 and S493, the super-resolution processing unit 206 adds the peak emphasizing images for each frequency group; performs different color coding for the image of each frequency group; and displays the color-coded image of each frequency group on the display device 108. In FIG. 12 is illustrated a diagrammatic form of the operations performed from Step S450 to Step S480. At Step S450, the data of N number of frames is subjected to Fourier transformation in the frame direction. At Step S460, the local maximum of the power values is extracted for each frequency. At Step S470, the local maximum of each frequency is added up. The region to be subjected to Fourier transformation is partially overlapped onto the region on the previous occasion.

Meanwhile, the super-resolution processing unit 206 can perform interpolation such as linear interpolation or spline interpolation with respect to the pixel count of the images of the image groups based on the characteristics of the bases or with respect to the pixel count of the original blood flow images of a plurality of frames, and then can extract the image values within a predetermined range or extract the area of the local maximum. That is done because, in a convex probe or a sector probe, the pixel size in the deep part is different than the pixel size in the shallow part; and because, if the pixels are coarse, even if the pixel values are extracted, the addition operation at Step S470 is performed, and the super-resolution processing is performed, the effect of resolution enhancement is only small. Meanwhile, in the case of performing linear processing, regardless of whether the processing is performed in the time axis or the frequency axis, the same effects are achieved. However, if nonlinear processing such as the local maximum is used, then the result in the time axis is different than the result in the frequency axis. In the case of Fourier transformation base, the local maximum is obtained for each blood flow velocity. Hence, it becomes easier to separate the blood vessels having different blood flow velocities and then extract the blood vessels.

As a result of performing the operation at Step S460, images as illustrated in FIG. 6C are obtained. Unlike the conventional technology, the super-resolution processing unit 206 extracts the image values from each image separated according to the blood velocity. As a result, not only the thick blood vessels having large image values, that is, having high blood flow velocity can be extracted, but the thin blood vessels having small image values, that is, having low blood flow velocity can also be extracted. That is, the super-resolution processing unit 206 becomes able to extract the blood vessels over a wide range of flow velocities. Moreover, in the case in which the energy level of the pixel values (for example, the standard deviation of the image values) of a particular blood vessel is close to the energy of the noise, since images separated according to the flow velocities are generated at Step S450, the noise energy gets dispersed among the bases thereby enabling achieving enhancement in the signal-to-noise ratio (SNR) or the contrast-to-noise ratio (CNR) of the images separated according to the flow velocities.

Hence, regarding the blood vessels that are not distinguishable in the conventional technology, the super-resolution processing unit 206 can extract such blood vessels. Moreover, as a result of dividing the images into a plurality of images at Step S450, the pixel values of each image become sparse; and, for example, in the operation performed at Step S460 to select the local peaks, as compared to the conventional technology, more pixels can be selected in the same number of frames. That enables shortening of the measurement time required to obtain the final image.

As explained above, at Step S460, the super-resolution processing unit 206 detects the local maximum points of an image of scalar values at each frequency, and emphasizes the scalar values of the local maximum points.

Among the images in an image group based on the characteristics, there is movement of the image values within a predetermined range or movement of the area having the local maximum. For that reason, the super-resolution processing unit 206 adds up the images of the characteristics-based image groups, in which the image values in a predetermined range are emphasized or the local maximum points are emphasized, across the character-based image groups and across the bases; generates a super-resolution blood flow image having enhanced resolution as illustrated in FIG. 6D; and displays that super-resolution blood flow image on the display device 108 (Step S480). Herein, the super-resolution processing unit 206 either can display, as the image on the display device 108, the result of adding up the images and the bases of all characteristics-based image groups; or can sequentially update intermediate images obtained by addition in chronological order, display the intermediate images on the display device 108, and as a result display a video on the display device 108; or can display the intermediate images side by side and without updating on the display device 108. Meanwhile, instead of adding the images and the bases of all characteristics-based image groups, the super-resolution processing unit 206 can add the images and the bases within the user-specified chronological range.

At the time of performing the addition, if the weight with respect to the image values of each base is kept uniform, the super-resolution processing unit 206 can extract the blood vessels having the flow velocity over a wide range. On the other hand, if the weight with respect to the image values of each base is varied, the super-resolution processing unit 206 can also emphasize the blood vessels having a particular flow velocity. For example, by increasing the weight of the base corresponding to the blood vessels having low flow velocity, the super-resolution processing unit 206 can emphasize the thin blood vessels having low flow velocity.

In this way, blood vessels having the flow velocity over a wide range can be extracted from the blood flow images of a plurality of frames, and a super-resolution blood flow image can be generated having enhanced resolution.

Given below is the explanation of a specific display method implemented at Step S480. Till Step S470, the power value is calculated by adding peak emphasizing images for each frequency. That is, in between the operation performed at Step S460 and the operation performed at Step S480, the peak emphasizing images are added for each frequency as the operation at Step S470, and the power value is calculated by adding the peak emphasizing images across all frequencies. Alternatively, at Step S490, the super-resolution processing unit 206 adds the peak emphasizing images for each positive frequency and adds the peak emphasizing images for each negative frequency. Still alternatively, at Step S492, the super-resolution processing unit 206 divides the frequencies into groups and adds the peak emphasizing values for each group.

In FIG. 10C is illustrated an image that is displayed by adding the power values, which are obtained at Step S470, across all frequencies. As illustrated in FIG. 6B, since the frequency and the flow velocity are comparable, by displaying a particular frequency, only a particular flow velocity can be displayed. Moreover, depending on the frequency, the color can also be varied at the time of display. For example, since a positive frequency represents the approaching blood flow, when an image formed by adding the positive frequencies is displayed on the display device 108, the super-resolution processing unit 206 can perform the super-resolution display of only the blood flow that is approaching the ultrasonic probe 102. Moreover, when an image formed by adding the negative frequencies is displayed on the display device 108, the super-resolution processing unit 206 can perform the super-resolution display of only the blood flow that is receding from the ultrasonic probe 102. Furthermore, by expressing the positive frequencies and the negative frequencies in different hues and then performing RGB addition, the super-resolution processing unit 206 can simultaneously express the approaching blood flow and the receding blood flow. Moreover, the super-resolution processing unit 206 can set a threshold value for the power of the approaching blood flow and can display, on the front, the signals having the power equal to or greater than the threshold value. As a result, the arteries can be displayed cleanly. Such conditions are illustrated in FIGS. 11A to 11H.

FIGS. 11A to 11H are diagrams illustrating the images of the kidney blood flow that are obtained according to various methods. In FIG. 11A is illustrated an image which is not subjected to super-resolution processing and in which the blood flow power is displayed in grayscale without super-resolution display. In FIG. 11B is illustrated an image which is not subjected to super-resolution processing and in which the blood flow power values are color-coded according to the directions. In the image illustrated in FIG. 11B, the approaching blood flow is expressed using red color, and the receding blood flow is expressed using blue color. At any single position, either the approaching blood flow can be expressed, or the receding blood flow can be expressed. In FIG. 11C is illustrated an image which is subjected to super-resolution processing on the time axis according to the conventional technology and in which the blood flow power values are displayed in grayscale. In FIG. 11D is illustrated an image which is subjected to super-resolution processing on the frequency axis according to the second embodiment and in which the power of all frequencies is added and displayed in grayscale. As compared to the image portion inside a circle drawn on the image illustrated in FIG. 11C, in the image portion inside a circle drawn on the image illustrated in FIG. 11D, it can be seen that the blood vessels are displayed with clean separation therebetween. In FIG. 11E is illustrated an image in which the power of only the positive frequencies (i.e., the approaching blood flow) is displayed. In the image illustrated in FIG. 11E, the approaching blood flow is expressed using red color. In FIG. 11F is illustrated an image in which the power of only the negative frequencies (i.e., the receding blood flow) is displayed. In the image illustrated in FIG. 11F, the receiving blood flow is expressed using blue color. In FIG. 11G is illustrated an image displayed as a result performing RGB addition of the image illustrated in FIG. 11E and the image illustrated in FIG. 11F. In FIG. 11H is illustrated an image in which the positive frequencies equal to or higher than a threshold value are displayed on the front. In the case of the kidneys, since the approaching blood flow represents the renal arteries and the receding blood flow represents the renal veins, the distinction between the arteries and the veins is achieved on the display. Thus, even when there is overlapping of blood vessels at a position, the arteries and the veins are separately displayed as illustrated in the images in FIGS. 11G and 11H. Moreover, it is also possible to display only the arteries as illustrated in the image in FIG. 11E, and it is also possible to display only the veins as illustrated in the image in FIG. 11F.

As explained above, at Steps S480, S491, and S493, from the frequency information and from scalar value integration images of all frequencies (i.e., images in which the local maximum point of the scalar values of a particular frequency is emphasized), the super-resolution processing unit 206 generates the third-type blood flow information having higher resolution than the resolution of the first-type blood flow information, and displays the third-type blood flow information on the display device 108. Moreover, the super-resolution processing unit 206 adds up a plurality of scalar value integration images of positive frequencies and calculates the blood flow information of the positive frequencies; adds up a plurality of scalar value integration images of negative frequencies and calculates the blood flow information of the negative frequencies; and displays the blood flow information of the positive frequencies and the blood flow information of the negative frequencies in a color-coded manner on the display device 108.

Moreover, at Step S491, the super-resolution processing unit 206 either displays, on the display device 108, a third-type RGB image that is formed by adding a first-type RGB image, which indicates the blood flow information of the positive frequencies, and a second-type RGB image, which indicates the blood flow information of the negative frequencies; or displays, on the display device 108, either a first-type RGB image or a second-type RGB image on the front with priority. Furthermore, the super-resolution processing unit 206 either adds up the scalar value integration images of a plurality of positive frequencies and calculates the blood flow information of the positive frequencies, or adds up the scalar value integration images of a plurality of negative frequencies and calculates the blood flow information of the negative frequencies; and either displays the blood flow information of the positive frequencies on the display device 108, or displays the blood flow information of the negative frequencies on the display device 108.

In the explanation given above, the super-resolution processing unit 206 divides the frequencies into two types, namely, the positive frequencies and the negative frequencies. However, alternatively, mutually different color-coding can be performed corresponding to all frequencies. Since each individual Doppler frequency corresponds to a particular flow velocity of the blood flow, the super-resolution processing unit 206 performs different color-coding according to the blood flow velocity. That is, the super-resolution processing unit 206 displays, on the display device 108, the scalar value integration image of each frequency in a different hue set for that frequency. Moreover, as the hue of a particular blood flow velocity, the super-resolution processing unit 206 can display luminance as the power of the blood flow having that particular flow velocity. Furthermore, when the centroid frequency (the first-order moment) of the power on the frequency axis is calculated, it represents the average velocity of the blood flow. The super-resolution processing unit 206 either can display only the average velocity or can display a two-dimensional colormap of the average velocity and the power. Moreover, when the second-order moment of the power on the frequency axis is calculated, the dispersion of the blood flow velocity is obtained. Thus, the super-resolution processing unit 206 can display the dispersion in combination with the flow velocity and the power. Moreover, the super-resolution processing unit 206 can create an image of the power values at each frequency included in the second-type blood flow information; can detect the local maximum of the image of the power values at each frequency; can integrate the local maximum at each frequency and calculate an image in which the local maximum points of the power values are emphasized; can calculate the average velocity and the dispersion of the blood flow from the images in which the local maximum point of the power values at a particular frequency is emphasized; and can display, on the display device 108, a multidimensional colormap of one or two or more of the average velocity, the dispersion, and the power value.

Till now, the explanation was given about the second embodiment. According to the second embodiment, it becomes possible to obtain blood flow information (blood flow images) in which the resolution is enhanced for the blood vessels having the flow velocity over a wide range or having various diameters and shapes. For example, according to the second embodiment, overlapped blood vessels too can be independently displayed with high resolution, and the information about the direction and the velocity of the blood flow can be displayed at the same time of displaying form information. Hence, according to the second embodiment, it becomes possible to obtain information having a high degree of user-friendliness.

Other embodiments

The technology disclosed herein can be implemented as an illustrative embodiment of a system, a device, a method, a computer program, or a recording medium (storage medium). More particularly, the technology disclosed herein can be applied in a system configured with a plurality of devices (such as a host computer, an interface device, an imaging device, and a web application); or can be applied in a single device. For example, the technology explained above can be applied in an image processing device. For example, in the memory of an image processing device, received signals that are obtained from an ultrasonic diagnostic apparatus and that include the data row of a plurality of frames in the time direction are stored in advance. That is, the memory is used to store the data row of reflected-wave data that is taken at one or more positions in a plurality of frames in the time direction by ultrasonic transmission and reception performed by the ultrasonic diagnostic apparatus. Then, the processor of the image processing device obtains the received signals (the data row of the reflected-wave data taken in a plurality of frames). Then, with respect to the received signals, the processor performs identical operations to the operations performed by the ultrasonic diagnostic apparatus as explained above.

Meanwhile, it goes without saying that the objective of the present invention is achieved in the following manner too. A system or a device is provided with a recording medium (or a storage medium) in which the program code of software (i.e., a computer program) meant for implementing the functions according to the embodiments described above is recorded. It goes without saying that such a storage medium is computer-readable. Then, a computer (or a CPU or an MPU) of the concerned system or the concerned device reads the program code from the recording medium and executes the program code. In that case, the program code that has been read from the recording medium implements the functions of the embodiments described above, and the recording medium in which the program code is recorded constitutes the present invention.

Meanwhile, the term "processor" used in the description of the embodiments implies, for example, a central processing unit (CPU), or a graphics processing unit (GPU), or an application specific integrated circuitry (ASIC), or a programmable logic device (such as a simple programmable logic device (SPLD), or a complex programmable logic device (CPLD), or a field programmable gate array (FPGA)). Moreover, instead of storing a computer program in the memory circuit 14, it can be directly incorporated into the circuitry of a processor. In that case, the processor reads the computer program incorporated in the circuitry and executes it so that the functions get implemented. Meanwhile, the processors according to the embodiments are not limited to be configured using a single circuitry on a processor-by-processor basis. Alternatively, a single processor can be configured by combining a plurality of independent circuitries, and the corresponding functions can be implemented.

A computer program executed by a processor is stored in advance in a read only memory (ROM) or a memory circuit. Alternatively, the computer program can be recorded as an installable file or an executable file in a non-transitory computer-readable recording medium such as a compact disk read only memory (CD-ROM), a flexible disk (FD), a compact disk recordable (CD-R), or a digital versatile disk (DVD). Still alternatively, the computer program can be stored in a downloadable manner in a computer that is connected to a network such as the Internet. For example, the computer program is configured using modules of the processing functions explained above. As far as the actual hardware is concerned, a CPU reads the computer program from a memory medium such as a ROM and executes it, so that the modules get loaded and generated in a main memory device.

Regarding the embodiments described above, the following notes are disclosed as an aspect of the invention and as the selective features.

(Note 1)

An ultrasonic diagnostic apparatus provided as an aspect of the present invention includes: an obtaining unit that obtains a data row of reflected-wave data that is taken at one or more positions in a plurality of frames in the time direction by performing ultrasonic transmission and reception; a Doppler processing unit that reduces the clutter component, which results from the tissues, from the data row of the plurality of frames and estimates first-type blood flow information; and a super-resolution processing unit that generates, from the first-type blood flow information, second-type blood flow information representing images of scalar values of blood flow signals, detects local maximums of the second-type blood flow information, integrates the local maximums or integrates images in which the local maximums are emphasized and generates a scalar value integration image, calculates an autocorrelation function in the frame direction at positions of the local maximum in the first-type blood flow information, integrates the autocorrelation functions and generates an autocorrelation function integration image, generates third-type blood flow information, in which the blood flow is color-coded, from the scalar value integration image and the autocorrelation function integration image, and displays the third-type blood flow information on a display unit.

(Note 2)

The super-resolution processing unit can calculate information about the direction of blood flow from the autocorrelation function integration image and can generate the third-type blood flow information, which is color-coded according to the direction of the blood flow, from the scalar value integration image and the information about the direction of the flow.

(Note 3)

The scalar values can represent the power values of the blood flow signals; and the super-resolution processing unit can detect the local maximums of the power values of the second-type blood flow information, can integrate the local maximum or integrate the images in which the local maximums are emphasized and generate a power value integration image as a scalar value integration image, can calculate the velocity and the dispersion of the blood flow from the power value integration image and the autocorrelation function integration image, and can perform color display of two or three sets of information from among the velocity, the dispersion, and the power value as the third-type blood flow information on the display unit.

(Note 4)

The Doppler processing unit can interpolate the first-type blood flow information, from which the clutter component resulting from the tissues is reduced, in the ultrasonic scanning line direction, and can increase the number of scanning lines.

(Note 5)

An image processing device provided as an aspect of the present invention includes: an obtaining unit that obtains a data row of reflected-wave data that is taken at one or more positions in a plurality of frames in the time direction by performing ultrasonic transmission and reception; a Doppler processing unit that reduces the clutter component, which results from the tissues, from the data row of the plurality of frames and estimates first-type blood flow information; and a super-resolution processing unit that generates, from the first-type blood flow information, second-type blood flow information representing images of scalar values of blood flow signals, detects local maximums of the second-type blood flow information, integrates the local maximums or integrates images in which the local maximums are emphasized and generates a scalar value integration image, calculates an autocorrelation function in the frame direction at positions of the local maximum in the first-type blood flow information, integrates the autocorrelation functions and generates an autocorrelation function integration image, generates third-type blood flow information, in which the blood flow is color-coded, from the scalar value integration image and the autocorrelation function integration image, and displays the third-type blood flow information on a display unit.

(Note 6)

An image processing method provided as an aspect of the present invention performs an operation of obtaining a data row of reflected-wave data that is taken at one or more positions in a plurality of frames in the time direction by performing ultrasonic transmission and reception; an operation of reducing the clutter component, which results from the tissues, from the data row of the plurality of frames and estimating first-type blood flow information; and an operation of generating, from the first-type blood flow information, second-type blood flow information representing images of scalar values of blood flow signals, detecting local maximums of the second-type blood flow information, integrating the local maximums or integrating images in which the local maximums are emphasized, and generating a scalar value integration image, calculating an autocorrelation function in the frame direction at positions of the local maximum in the first-type blood flow information, integrating the autocorrelation functions and generating an autocorrelation function integration image, generating third-type blood flow information, in which the blood flow is color-coded, from the scalar value integration image and the autocorrelation function integration image, and displaying the third-type blood flow information on a display unit.

(Note 7)

An ultrasonic diagnostic apparatus provided as an aspect of the present invention includes: an obtaining unit that obtains a data row of reflected-wave data that is taken at one or more positions in a plurality of frames in the time direction by performing ultrasonic transmission and reception; a Doppler processing unit that reduces the clutter component, which results from the tissues, from the data row of the plurality of frames and estimates first-type blood flow information; and a super-resolution processing unit that generates, from the first-type blood flow information, second-type blood flow information representing images of scalar values of blood flow signals, detects local maximums of the second-type blood flow information, integrates the local maximums or integrates images in which the local maximums are emphasized and generates a scalar value integration image, generates third-type blood flow information, in which blood flow is color-coded, from the scalar value integration image and information related to the direction of the blood flow at the positions corresponding to the local maximums, and displays the third-type blood flow information on a display unit.

(Note 8)

An ultrasonic diagnostic apparatus provided as another aspect of the present invention includes: an obtaining unit that obtains reflected-wave data that is taken at one or more positions in a plurality of frames in the time direction by performing ultrasonic transmission and reception; a Doppler processing unit that reduces the clutter component, which results from the tissues, from a data row of the plurality of frames and estimates first-type blood flow information; and a super-resolution processing unit that performs orthogonal transformation in the frame direction and calculates second-type blood flow information from the first-type blood flow information, creates a scalar value image for each frequency of the second-type blood flow information, detects the local maximum point in the image of scalar values at each frequency and performs an operation to emphasize the scalar values of the local maximum points, generates third-type blood flow information from frequency information and from the image in which the local maximum point of the scalar values at each frequency is emphasized, and displays the third-type blood flow information on a display unit.

(Note 9)

The super-resolution processing unit can add up images in which the local maximums of a plurality of scalar values of positive frequencies are emphasized and calculate blood flow information of positive frequencies, can add up images in which the local maximums of scalar values of negative frequencies are emphasized and calculate blood flow information of negative frequencies, and can display the blood flow information of the positive frequencies and the blood flow information of the negative frequencies in a color-coded manner on the display unit.

(Note 10)

The super-resolution processing unit can either display, on the display unit, a third-type RGB image that is formed by adding a first-type RGB image, which indicates the blood flow information of the positive frequencies, and a second-type RGB image, which indicates the blood flow information of the negative frequencies; or display, on the display unit, either the first-type RGB image or the second-type RGB image on the front with priority.

(Note 11)

The super-resolution processing unit either can add up images in which the local maximums of a plurality of scalar values of positive frequencies are emphasized and calculate blood flow information of positive frequencies or can add up images in which the local maximums of scalar values of negative frequencies are emphasized and calculate blood flow information of negative frequencies, and either can display the blood flow information of the positive frequencies on the display unit or can display the blood flow information of the negative frequencies on the display unit.

(Note 12)

The super-resolution processing unit can display, on the display unit and with a different hue for each frequency, images in which local maximums of scalar values at each frequency are emphasized.

(Note 13)

The scalar values can represent the power values of the blood flow; and the super-resolution processing unit can create an image of the power values at each frequency included in the second-type blood flow information, detect the local maximum of the image of the power values at each frequency, integrate the local maximum at each frequency and calculate an image in which the local maximum points of the power values are emphasized, calculate the average velocity and the dispersion of the blood flow from the images in which the local maximum point of the power values at each frequency is emphasized, and display, on the display unit, a multidimensional colormap of one or two or more of the average velocity, the dispersion, and the power value.

(Note 14)

The Doppler processing unit can interpolate the first-type blood flow information, from which the clutter component resulting from the tissues is reduced, in the ultrasonic scanning line direction, and can increase the number of scanning lines.

(Note 15)

An image processing device provided as another aspect of the present invention includes: an obtaining unit that obtains reflected-wave data that is taken at one or more positions in a plurality of frames in the time direction by performing ultrasonic transmission and reception; a Doppler processing unit that reduces the clutter component, which results from the tissues, from a data row of the plurality of frames and estimates first-type blood flow information; and a super-resolution processing unit that performs orthogonal transformation in the frame direction and calculates second-type blood flow information from the first-type blood flow information, creates a scalar value image for each frequency of the second-type blood flow information, detects the local maximum point in the image of scalar values at each frequency and performs an operation to emphasize the scalar values of the local maximum points, generates third-type blood flow information from frequency information and from the image in which the local maximum point of the scalar values at each frequency is emphasized, and displays the third-type blood flow information on a display unit.

(Note 16)

An image processing method provided as another aspect of the present invention includes: an operation of obtaining reflected-wave data that is taken at one or more positions in a plurality of frames in the time direction by performing ultrasonic transmission and reception; an operation of reducing the clutter component, which results from the tissues, from a data row of the plurality of frames and estimating first-type blood flow information; an operation of performing orthogonal transformation in the frame direction and calculating second-type blood flow information from the first-type blood flow information; an operation of creating an image of scalar values for each frequency of the second-type blood flow information, detecting the local maximum point in the image of scalar values at each frequency, and performing an operation to emphasize the scalar values of the local maximum points; and an operation of generating third-type blood flow information from frequency information and from the image in which the local maximum point of the scalar values at each frequency is emphasized, and displaying the third-type blood flow information on a display unit.

According to at least one of the embodiments described above, it becomes possible to obtain information having a high degree of user-friendliness.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An ultrasonic diagnostic apparatus comprising processing circuitry that

   obtains a data row of reflected-wave data that is taken at one or more positions in a plurality of frames in time direction by performing ultrasonic transmission and reception,
   reduces clutter component, which results from tissue, from the data row of the plurality of frames and estimates first-type blood flow information,
   generates, from the first-type blood flow information, second-type blood flow information representing images of scalar values of blood flow signals, detects local maximums of the second-type blood flow information, integrates the local maximums or integrates images in which the local maximums are emphasized and generates a scalar value integration image, calculates an autocorrelation function in frame direction at positions of the local maximum in the first-type blood flow information, integrates the autocorrelation functions and generates an autocorrelation function integration image, generates third-type blood flow information, in which blood flow is color-coded, from the scalar value integration image and the autocorrelation function integration image, and displays the third-type blood flow information on a display.

2. The ultrasonic diagnostic apparatus according to claim 1, wherein the processing circuitry calculates information about direction of blood flow from the autocorrelation function integration image and generates the third-type blood flow information, which is color-coded according to the direction of the blood flow, from the scalar value integration image and information about the direction of the flow.

3. The ultrasonic diagnostic apparatus according to claim 1, wherein

   the scalar values represent power values of the blood flow signals, and
   the processing circuitry detects the local maximums of the power values of the second-type blood flow information, integrates the local maximum or integrates images in which the local maximums are emphasized and generates a power value integration image as a scalar value integration image, calculates velocity and dispersion of blood flow from the power value integration image and the autocorrelation function integration image, and performs color display of two or three sets of information from among the velocity, the dispersion, and the power value as the third-type blood flow information on the display.

4. The ultrasonic diagnostic apparatus according to claim 1, wherein the processing circuitry interpolates the first-type blood flow information, from which clutter component resulting from the tissue is reduced, in ultrasonic scanning line direction, and increases number of scanning lines.

5. An information processing method comprising:

   obtaining a data row of reflected-wave data that is taken at one or more positions in a plurality of frames in time direction by performing ultrasonic transmission and reception;
   estimating that includes reducing clutter component, which results from tissue, from the data row of the plurality of frames and estimating first-type blood flow information;
   generating, from the first-type blood flow information, second-type blood flow information representing images of scalar values of blood flow signals;
   generating that includes detecting local maximums of the second-type blood flow information, integrating the local maximums or integrating images in which the local maximums are emphasized, and generating a scalar value integration image;
   generating that includes calculating an autocorrelation function in frame direction at positions of the local maximum in the first-type blood flow information, integrating the autocorrelation functions, and generating an autocorrelation function integration image;
   generating third-type blood flow information, in which blood flow is color-coded, from the scalar value integration image and the autocorrelation function integration image; and
   displaying the third-type blood flow information on a display.

6. An ultrasonic diagnostic apparatus comprising processing circuitry that

   obtains a data row of reflected-wave data that is taken at one or more positions in a plurality of frames in time direction by performing ultrasonic transmission and reception,

reduces clutter component, which results from tissue, from the data row of the plurality of frames and estimates first-type blood flow information, and

generates, from the first-type blood flow information, second-type blood flow information representing images of scalar values of blood flow signals, detects local maximums of the second-type blood flow information, integrates the local maximums or integrates images in which the local maximums are emphasized and generates a scalar value integration image, generates third-type blood flow information, in which blood flow is color-coded, from the scalar value integration image and information related to direction of blood flow at positions corresponding to the local maximums, and displays the third-type blood flow information on a display.

**7.** An ultrasonic diagnostic apparatus comprising processing circuitry that

obtains reflected-wave data that is taken at one or more positions in a plurality of frames in time direction by performing ultrasonic transmission and reception,

reduces clutter component, which results from tissue, from a data row of the plurality of frames and estimates first-type blood flow information, and

performs orthogonal transformation in frame direction and calculates second-type blood flow information from the first-type blood flow information, creates a scalar value image for each frequency of the second-type blood flow information, detects local maximum point in the image of scalar values at each frequency and performs an operation to emphasize scalar values of local maximum points, generates third-type blood flow information from frequency information and from an image in which local maximum point of scalar values at each frequency is emphasized, and displays the third-type blood flow information on a display.

**8.** The ultrasonic diagnostic apparatus according to claim 7, wherein the processing circuitry adds up images in which local maximums of a plurality of scalar values of positive frequencies are emphasized and calculates blood flow information of positive frequencies, adds up images in which local maximums of a plurality of scalar values of negative frequencies are emphasized and calculates blood flow information of negative frequencies, and displays the blood flow information of the positive frequencies and the blood flow information of the negative frequencies in a color-coded manner on the display.

**9.** The ultrasonic diagnostic apparatus according to claim 8, wherein the processing circuitry either displays, on the display, a third-type RGB image that is formed by adding a first-type RGB image, which indicates the blood flow information of the positive frequencies, and a second-type RGB image, which indicates the blood flow information of the negative frequencies or displays, on the display, either the first-type RGB image or the second-type RGB image on front with priority.

**10.** The ultrasonic diagnostic apparatus according to claim 7, wherein the processing circuitry

either adds up images in which local maximums of a plurality of scalar values of positive frequencies are emphasized and calculates blood flow information of positive frequencies or adds up images in which local maximums of a plurality of scalar values of negative frequencies are emphasized and calculates blood flow information of negative frequencies, and

either displays the blood flow information of the positive frequencies on the display or displays the blood flow information of the negative frequencies on the display.

**11.** The ultrasonic diagnostic apparatus according to claim 7, wherein the processing circuitry displays, on the display and with a different hue for each frequency, images in which local maximums of scalar values at each frequency are emphasized.

**12.** The ultrasonic diagnostic apparatus according to claim 7, wherein

the scalar values represent power values of blood flow, and

the processing circuitry creates an image of the power values at each frequency included in the second-type blood flow information, detects local maximum of the image of the power values at each frequency, integrates the local maximum at each frequency and calculates an image in which local maximum points of power values are emphasized, calculates average velocity and dispersion of blood flow from images in which local maximum point of power values at each frequency is emphasized, and displays, on the display, a multidimensional colormap of one or two or more of the average velocity, the dispersion, and the power value.

**13.** The ultrasonic diagnostic apparatus according to claim 7, wherein the processing circuitry interpolates the first-type blood flow information, from which the clutter component resulting from the tissue is reduced, in ultrasonic scanning line direction, and increases number of scanning lines.

**14.** An image processing method comprising:

obtaining reflected-wave data that is taken at one or more positions in a plurality of frames in time direction by performing ultrasonic transmission and reception;
estimating that includes reducing clutter component, which results from tissue, from a data row of the plurality of frames and estimating first-type blood flow information;
calculating that includes performing orthogonal transformation in frame direction and calculating second-type blood flow information from the first-type blood flow information;
emphasizing that includes creating an image of scalar values for each frequency of the second-type blood flow information, detecting local maximum point in the image of scalar values at each frequency, and emphasizing scalar values of local maximum points; and
displaying that includes generating third-type blood flow information from frequency information and from the image in which local maximum point of scalar values at each frequency is emphasized, and displaying the third-type blood flow information on a display.

FIG.1

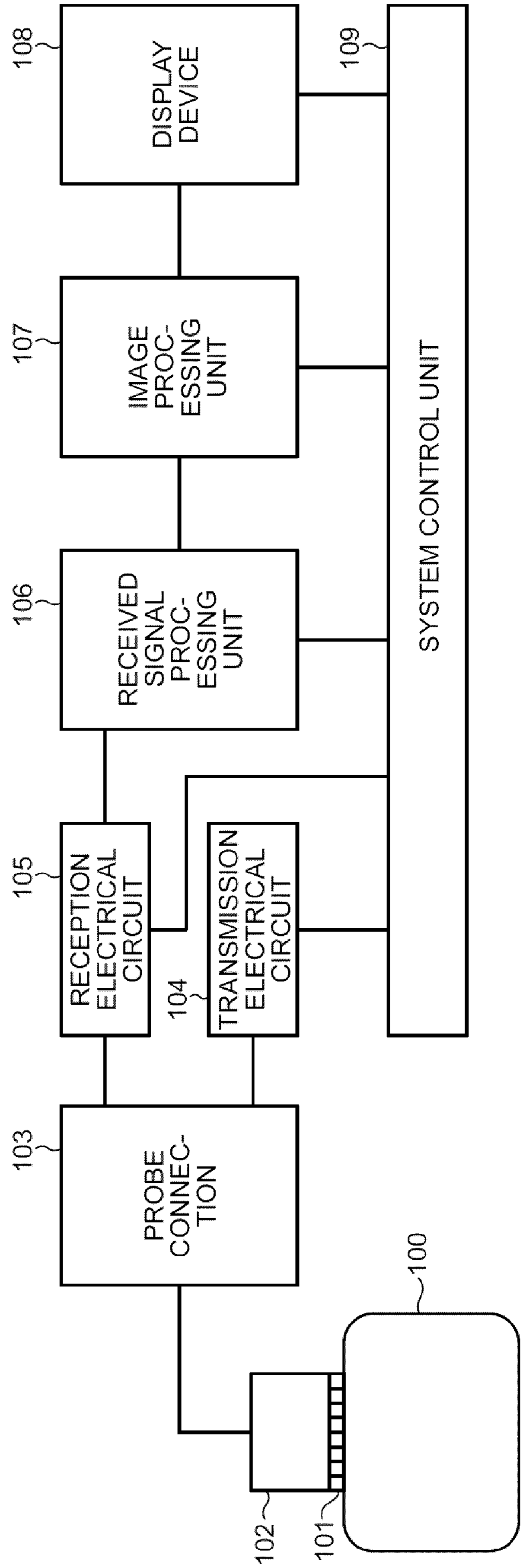
100
101
102
PROBE CONNECTION
103
TRANSMISSION ELECTRICAL CIRCUIT
104
RECEPTION ELECTRICAL CIRCUIT
105
RECEIVED SIGNAL PROCESSING UNIT
106
IMAGE PROCESSING UNIT
107
DISPLAY DEVICE
108
SYSTEM CONTROL UNIT
109

FIG.2

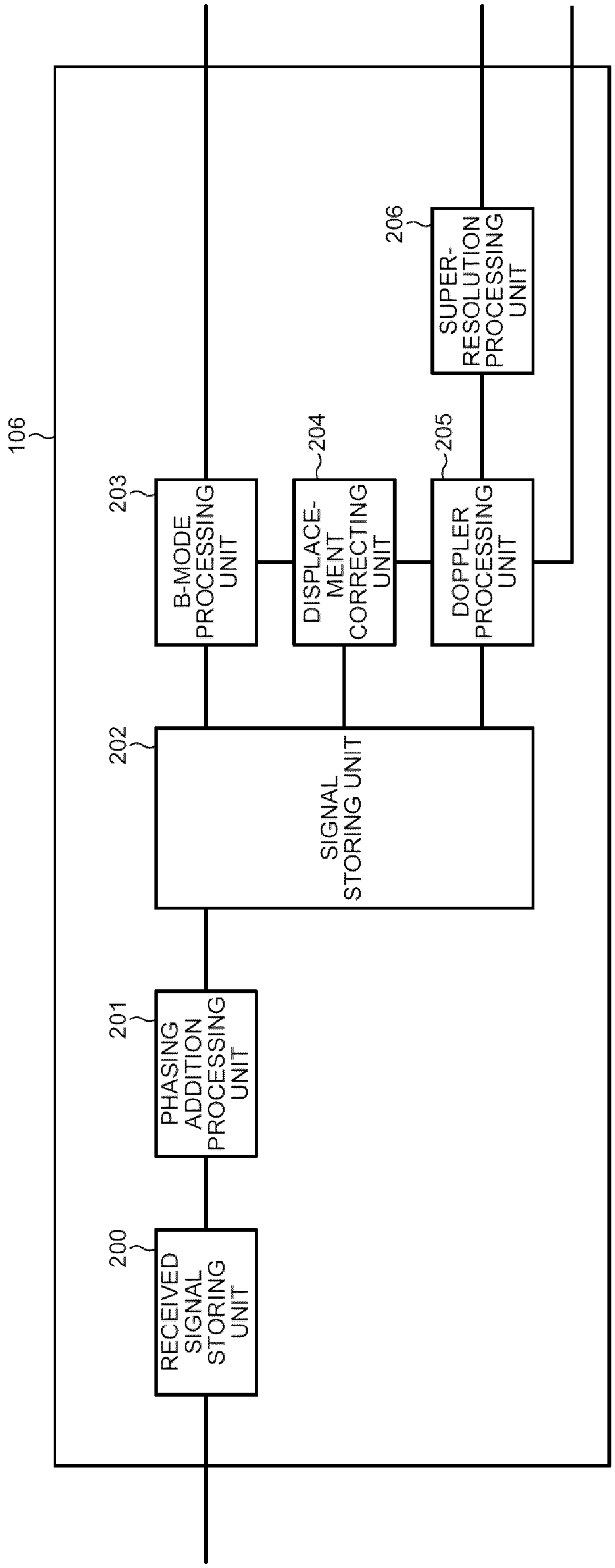
106
200
RECEIVED SIGNAL STORING UNIT
201
PHASING ADDITION PROCESSING UNIT
202
SIGNAL STORING UNIT
203
B-MODE PROCESSING UNIT
204
DISPLACE-MENT CORRECTING UNIT
205
DOPPLER PROCESSING UNIT
206
SUPER-RESOLUTION PROCESSING UNIT

FIG.3

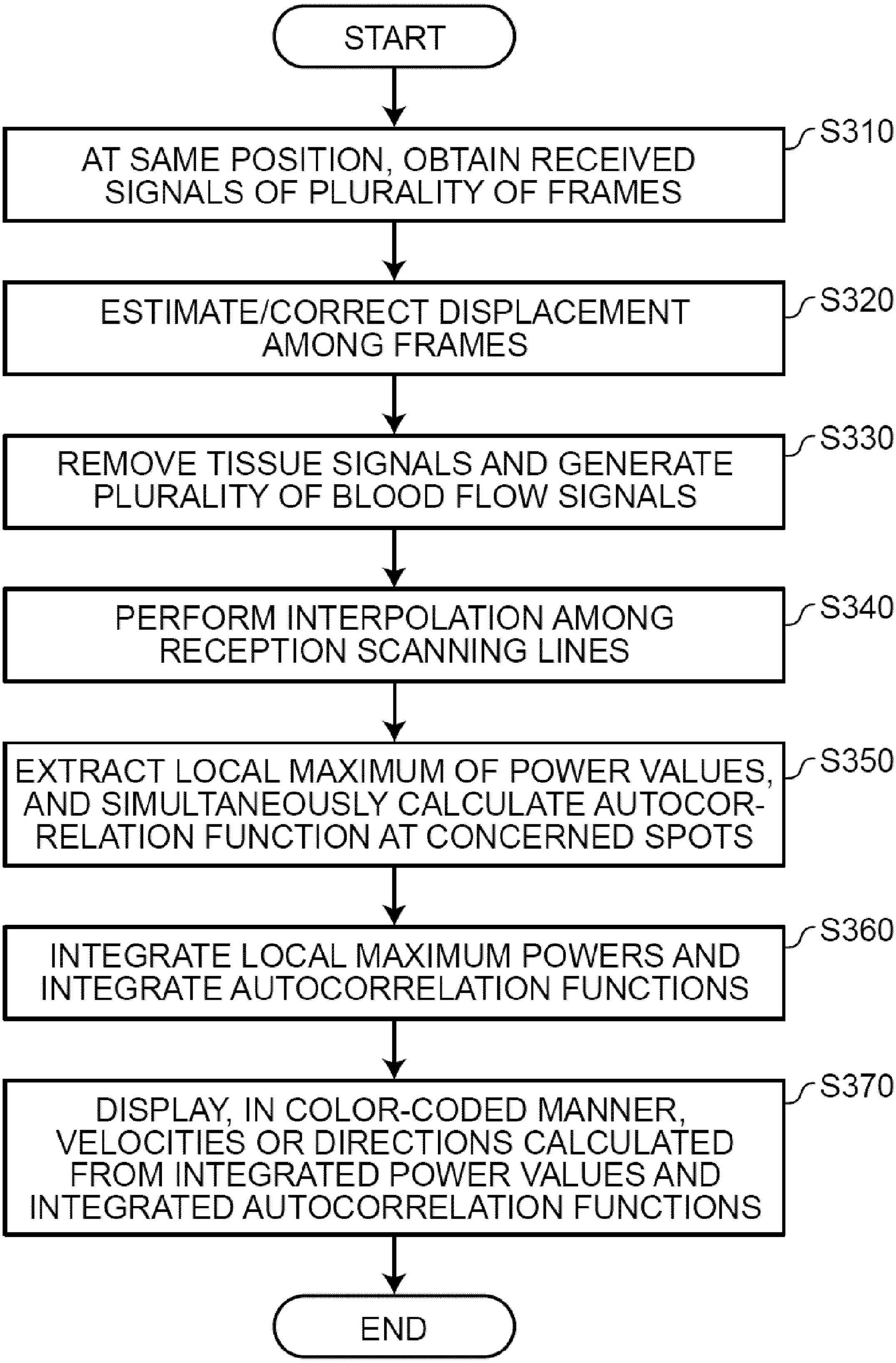
START
AT SAME POSITION, OBTAIN RECEIVED SIGNALS OF PLURALITY OF FRAMES
S310
ESTIMATE/CORRECT DISPLACEMENT AMONG FRAMES
S320
REMOVE TISSUE SIGNALS AND GENERATE PLURALITY OF BLOOD FLOW SIGNALS
S330
PERFORM INTERPOLATION AMONG RECEPTION SCANNING LINES
S340
EXTRACT LOCAL MAXIMUM OF POWER VALUES, AND SIMULTANEOUSLY CALCULATE AUTOCORRELATION FUNCTION AT CONCERNED SPOTS
S350
INTEGRATE LOCAL MAXIMUM POWERS AND INTEGRATE AUTOCORRELATION FUNCTIONS
S360
DISPLAY, IN COLOR-CODED MANNER, VELOCITIES OR DIRECTIONS CALCULATED FROM INTEGRATED POWER VALUES AND INTEGRATED AUTOCORRELATION FUNCTIONS
S370
END

FIG.4A

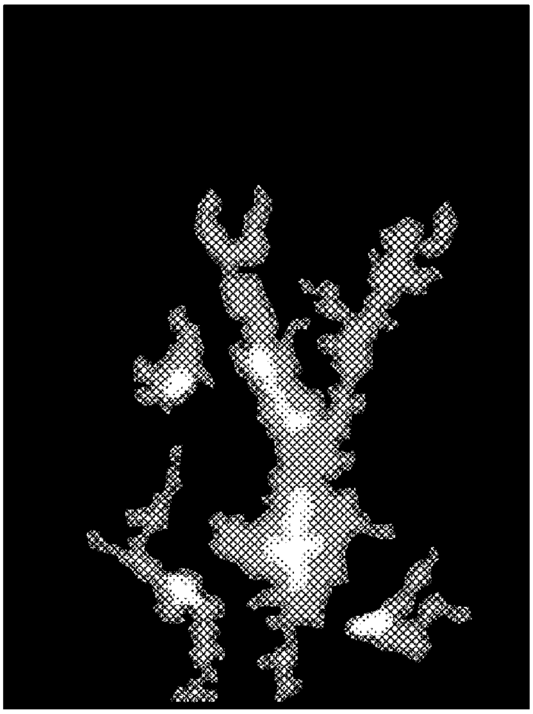

FIG.4B

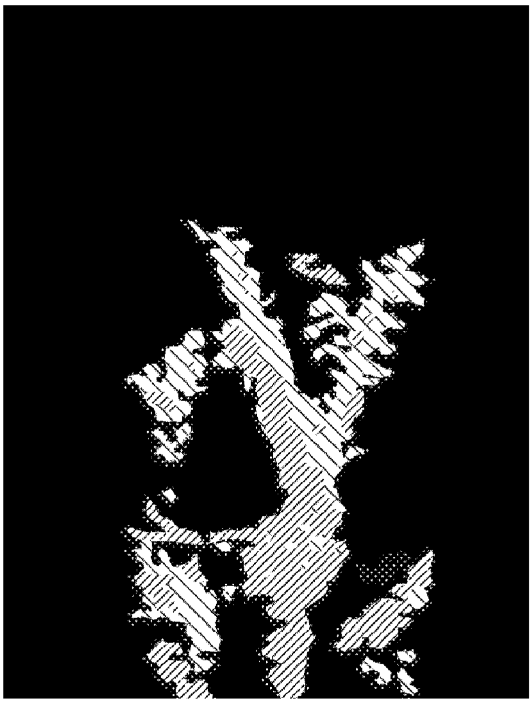

FIG.4C

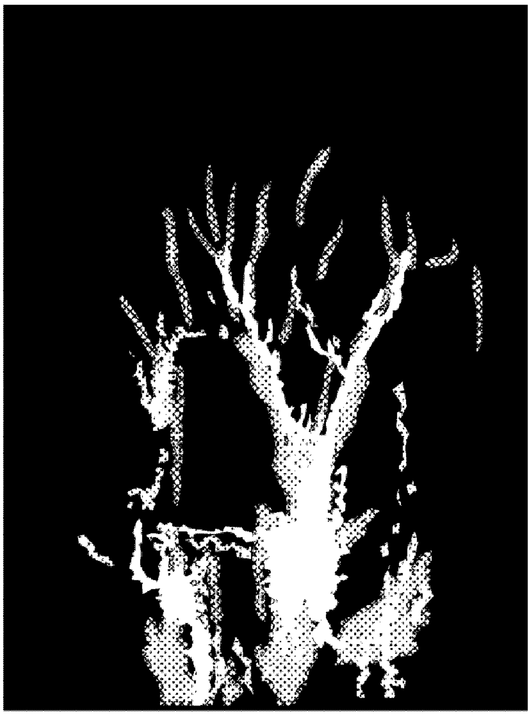

FIG.4D

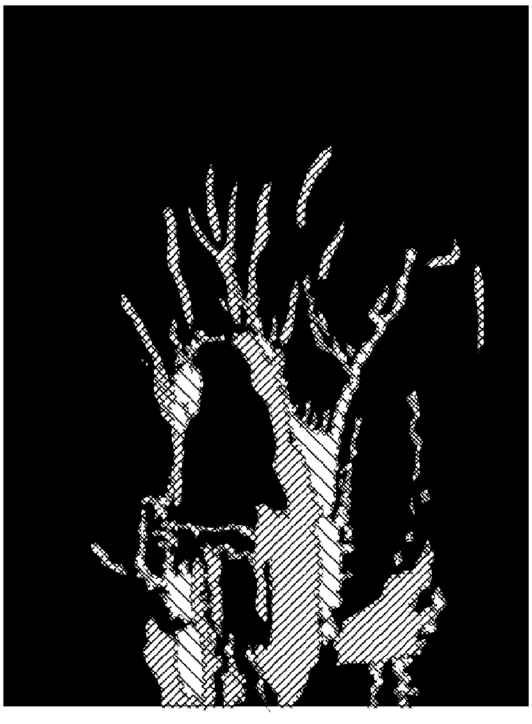

FIG.5

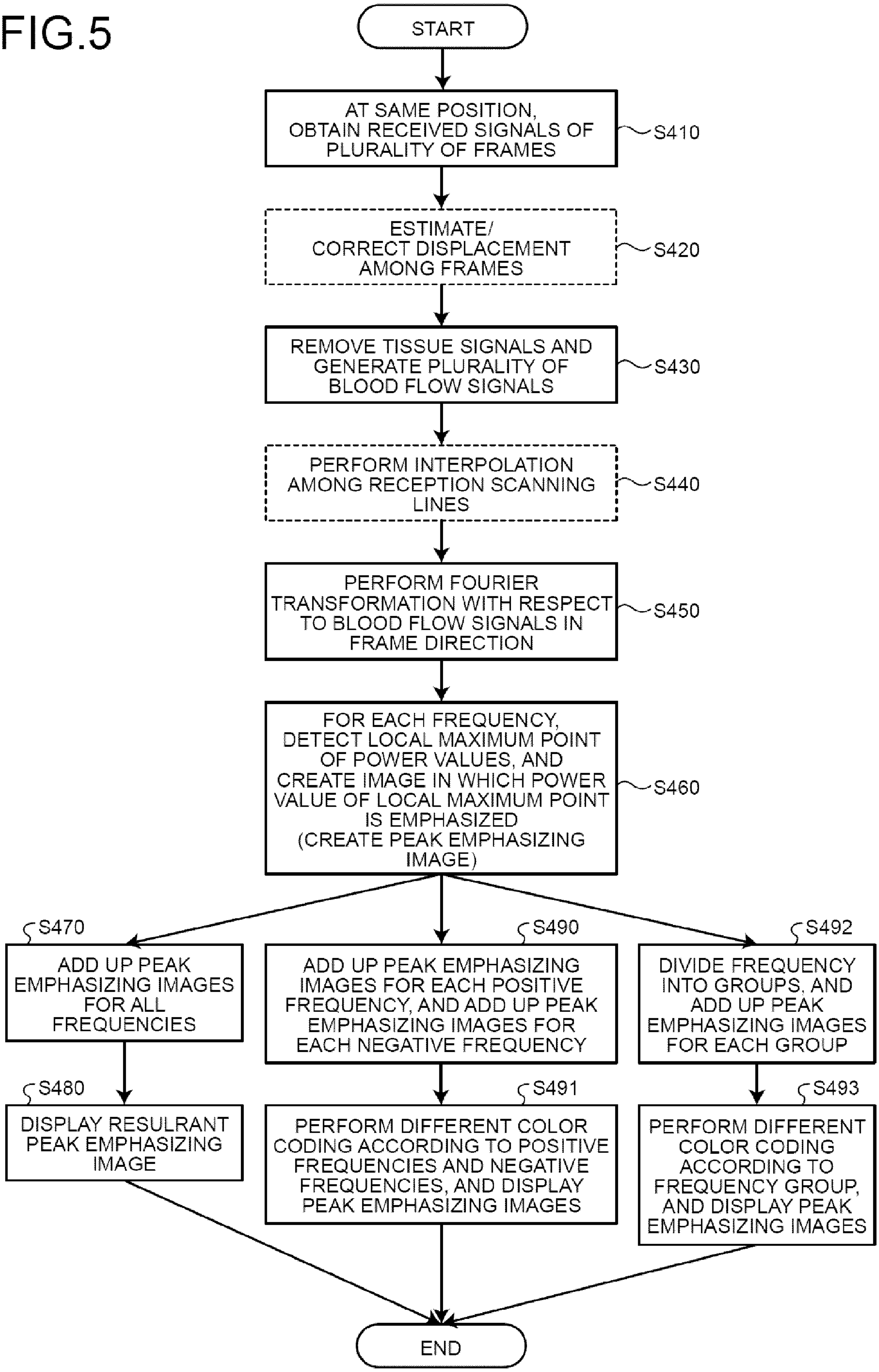
START
AT SAME POSITION, OBTAIN RECEIVED SIGNALS OF PLURALITY OF FRAMES
S410
ESTIMATE/ CORRECT DISPLACEMENT AMONG FRAMES
S420
REMOVE TISSUE SIGNALS AND GENERATE PLURALITY OF BLOOD FLOW SIGNALS
S430
PERFORM INTERPOLATION AMONG RECEPTION SCANNING LINES
S440
PERFORM FOURIER TRANSFORMATION WITH RESPECT TO BLOOD FLOW SIGNALS IN FRAME DIRECTION
S450
FOR EACH FREQUENCY, DETECT LOCAL MAXIMUM POINT OF POWER VALUES, AND CREATE IMAGE IN WHICH POWER VALUE OF LOCAL MAXIMUM POINT IS EMPHASIZED (CREATE PEAK EMPHASIZING IMAGE)
S460
S470
ADD UP PEAK EMPHASIZING IMAGES FOR ALL FREQUENCIES
S490
ADD UP PEAK EMPHASIZING IMAGES FOR EACH POSITIVE FREQUENCY, AND ADD UP PEAK EMPHASIZING IMAGES FOR EACH NEGATIVE FREQUENCY
S492
DIVIDE FREQUENCY INTO GROUPS, AND ADD UP PEAK EMPHASIZING IMAGES FOR EACH GROUP
S480
DISPLAY RESULRANT PEAK EMPHASIZING IMAGE
S491
PERFORM DIFFERENT COLOR CODING ACCORDING TO POSITIVE FREQUENCIES AND NEGATIVE FREQUENCIES, AND DISPLAY PEAK EMPHASIZING IMAGES
S493
PERFORM DIFFERENT COLOR CODING ACCORDING TO FREQUENCY GROUP, AND DISPLAY PEAK EMPHASIZING IMAGES
END

FIG.6A

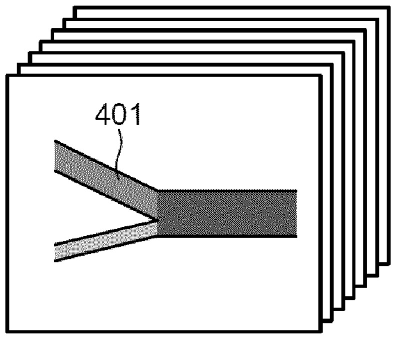


FIG.6B

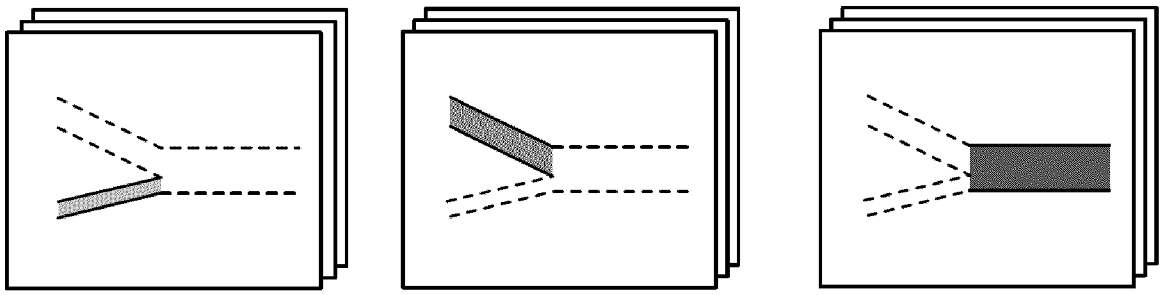

FIG.6C

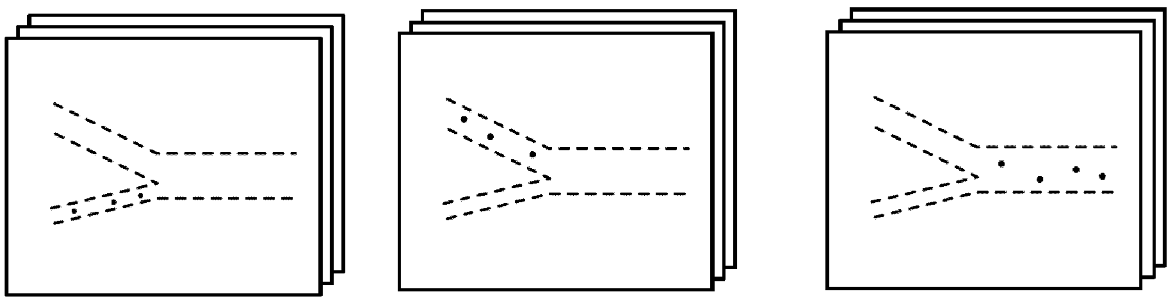

FIG.6D

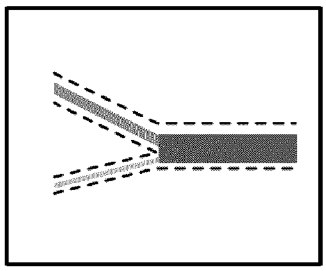

FIG.7

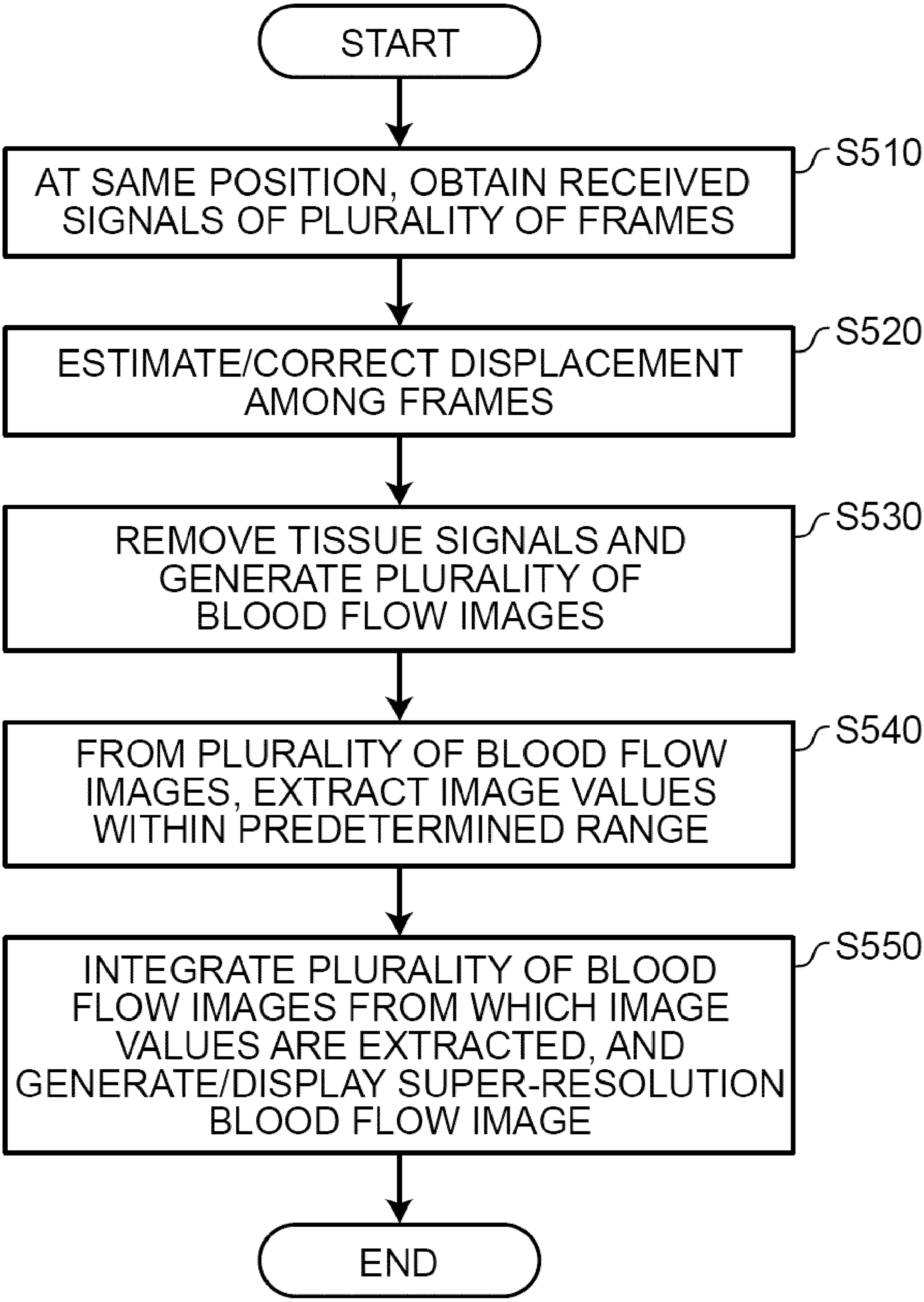
START
S510
AT SAME POSITION, OBTAIN RECEIVED SIGNALS OF PLURALITY OF FRAMES
S520
ESTIMATE/CORRECT DISPLACEMENT AMONG FRAMES
S530
REMOVE TISSUE SIGNALS AND GENERATE PLURALITY OF BLOOD FLOW IMAGES
S540
FROM PLURALITY OF BLOOD FLOW IMAGES, EXTRACT IMAGE VALUES WITHIN PREDETERMINED RANGE
S550
INTEGRATE PLURALITY OF BLOOD FLOW IMAGES FROM WHICH IMAGE VALUES ARE EXTRACTED, AND GENERATE/DISPLAY SUPER-RESOLUTION BLOOD FLOW IMAGE
END

FIG.8A

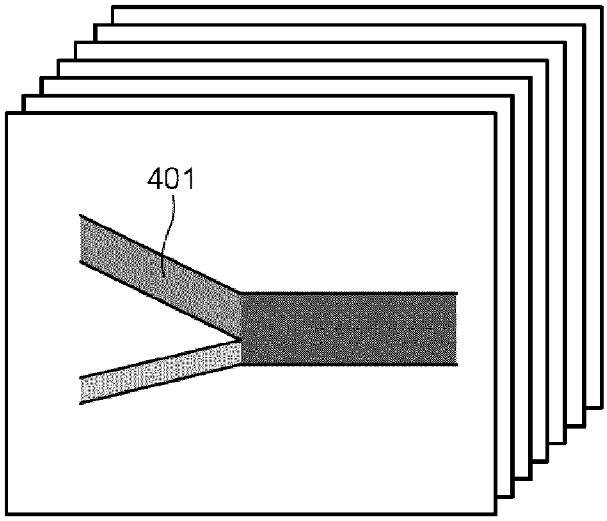


FIG.8B

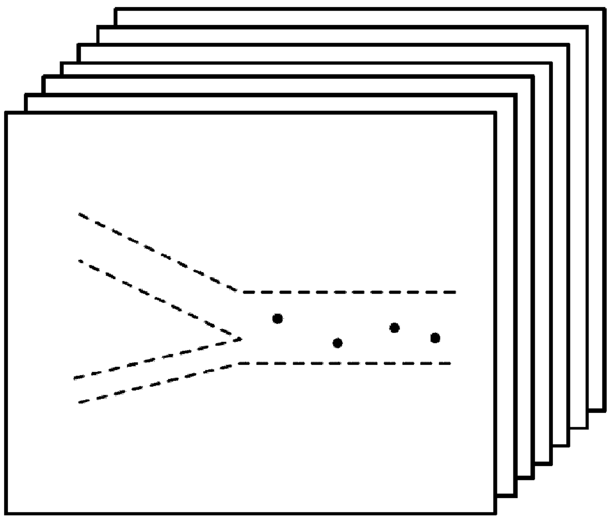

FIG.8C

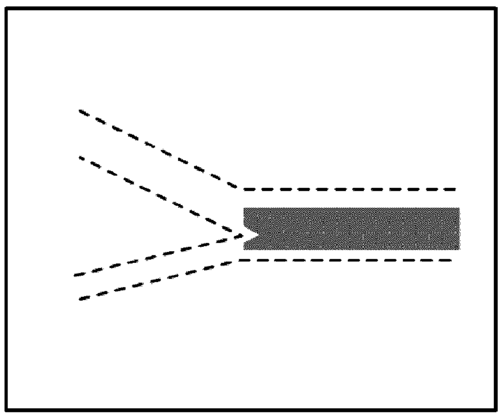

FIG.9

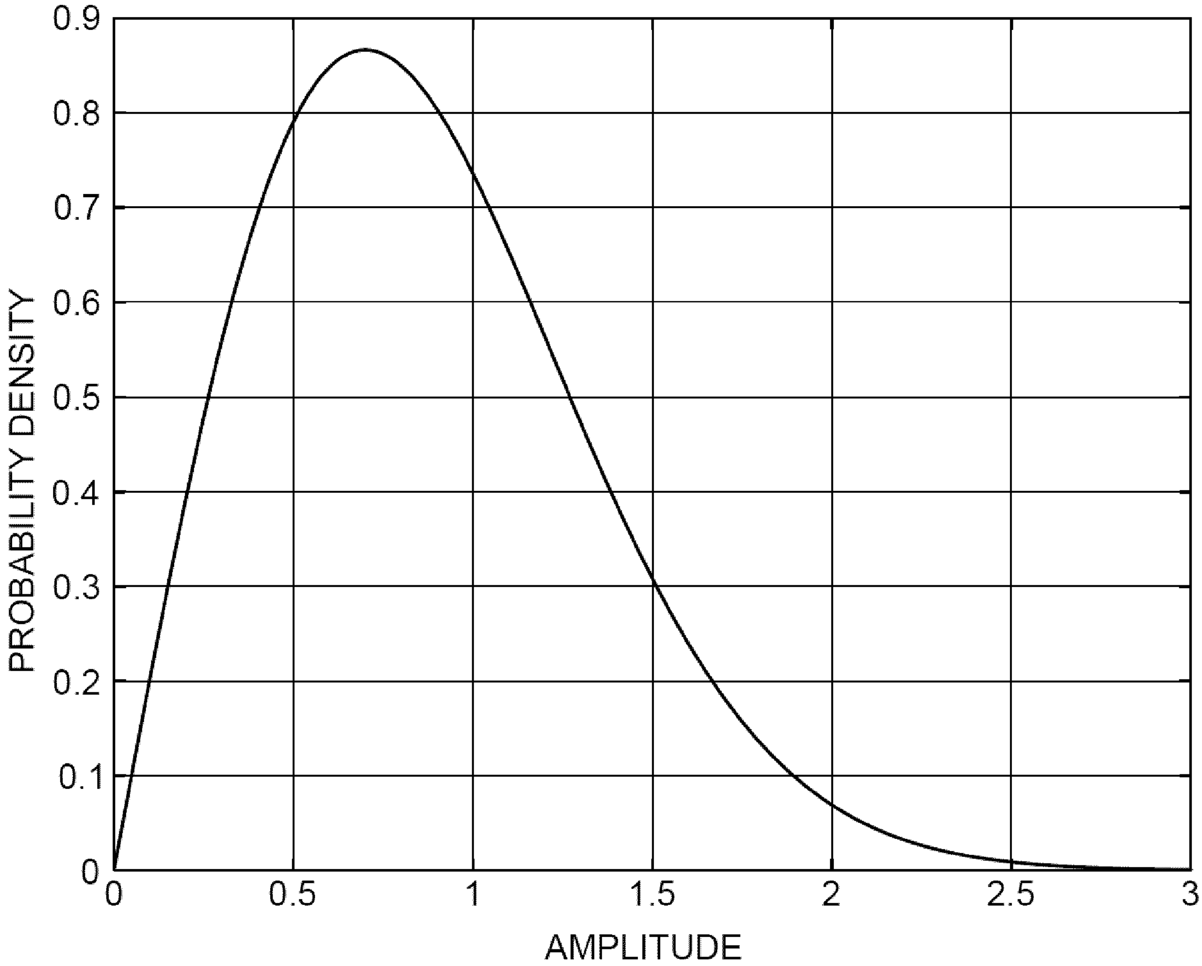
0.9
0.8
0.7
0.6
0.5
0.4
0.3
0.2
0.1
0
PROBABILITY DENSITY
0
0.5
1
1.5
2
2.5
3
AMPLITUDE

FIG.10A

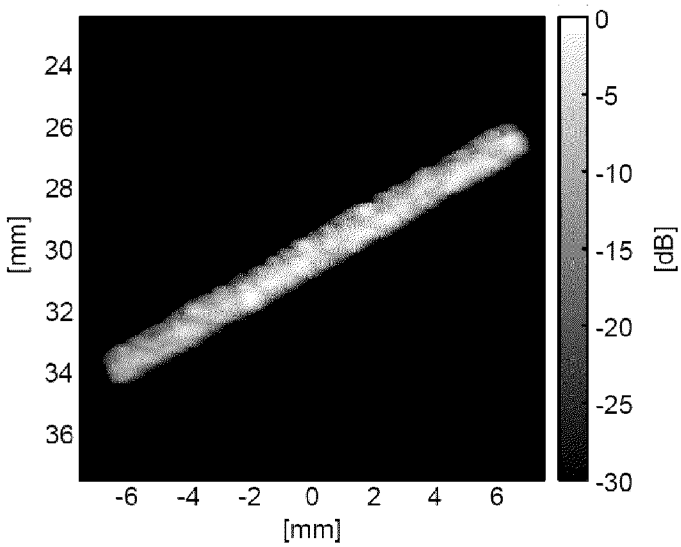


FIG.10B

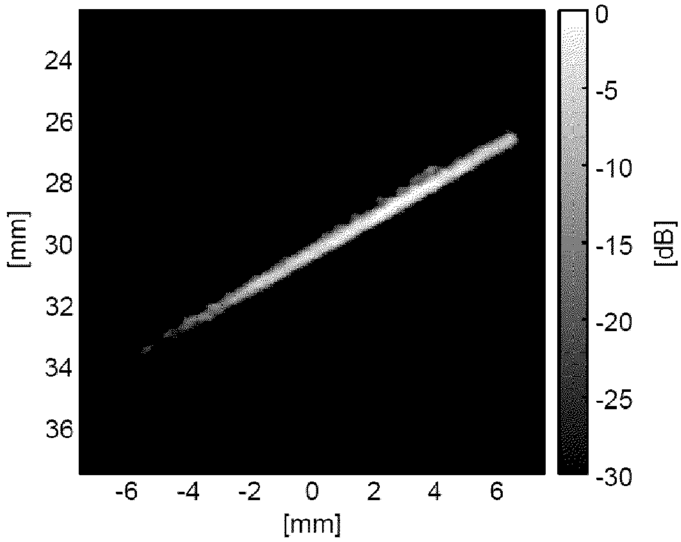


FIG.10C

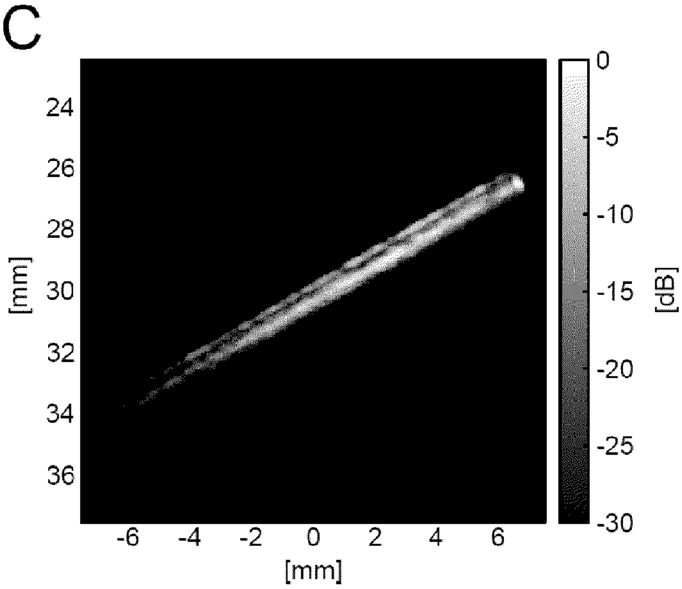

FIG.11A

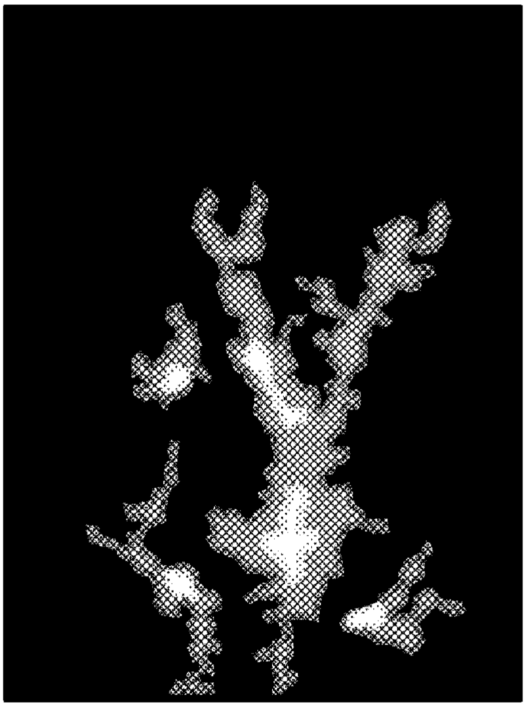

FIG.11B

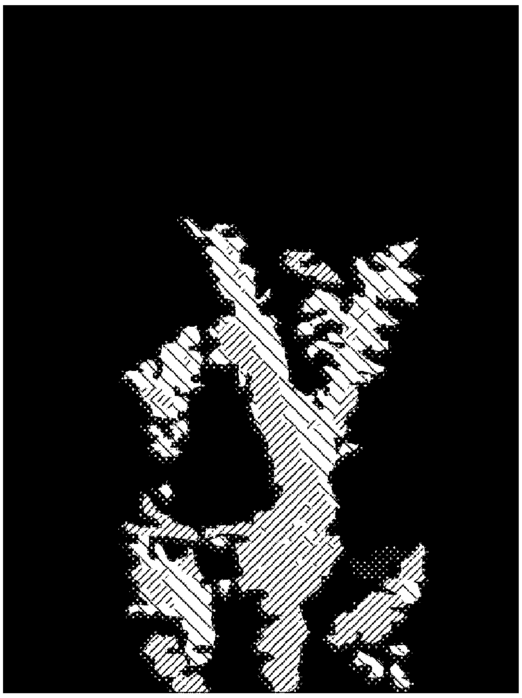

FIG.11C

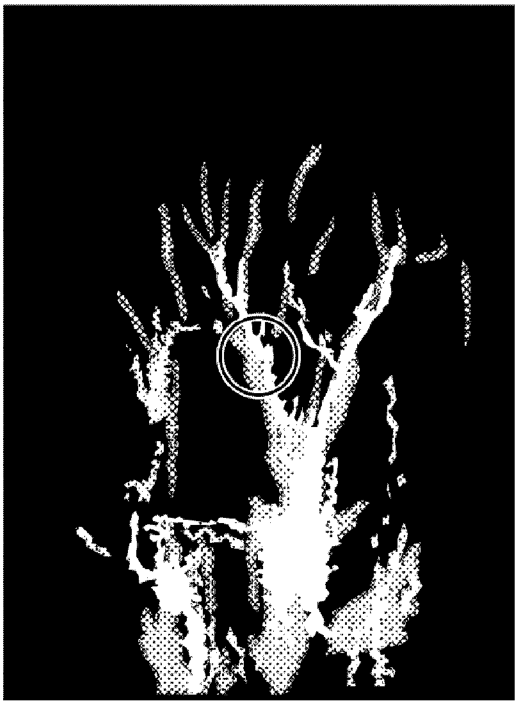

FIG.11D

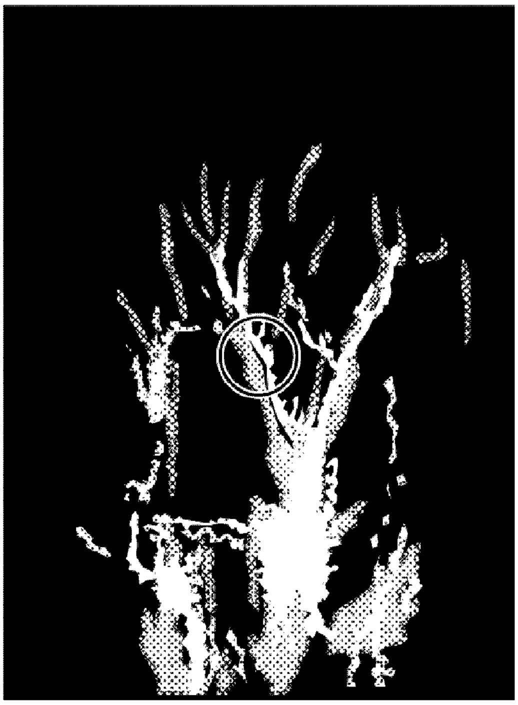

FIG.11E

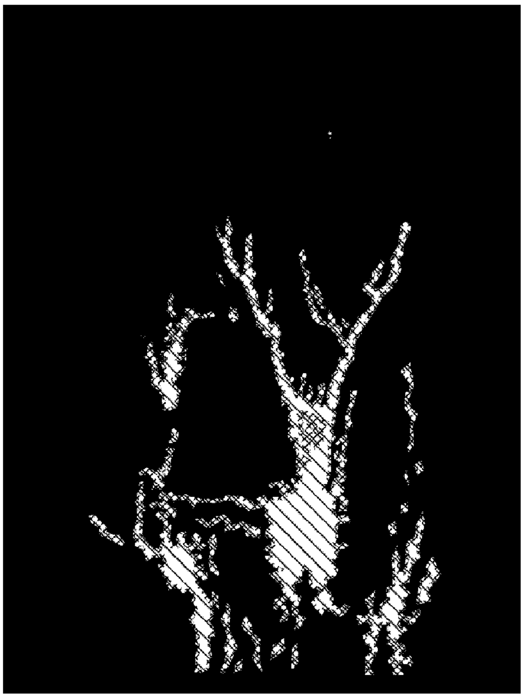

FIG.11F

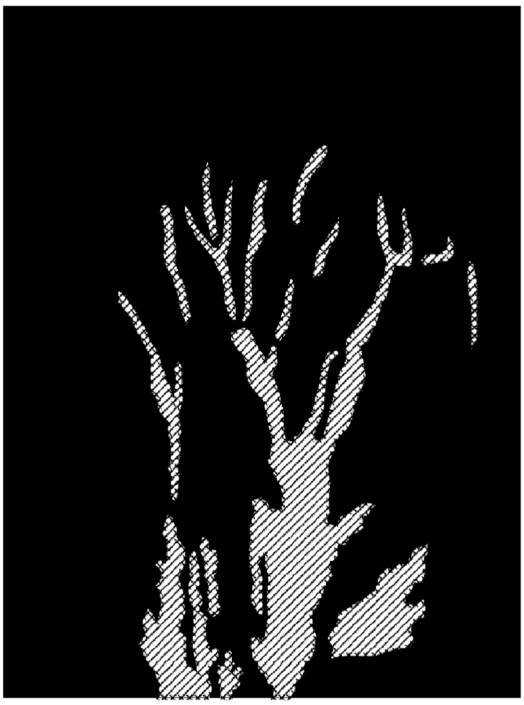

FIG.11G

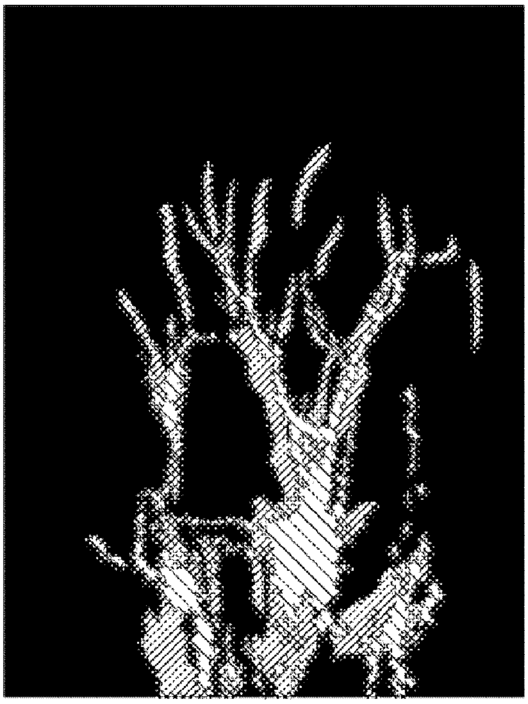

FIG.11H

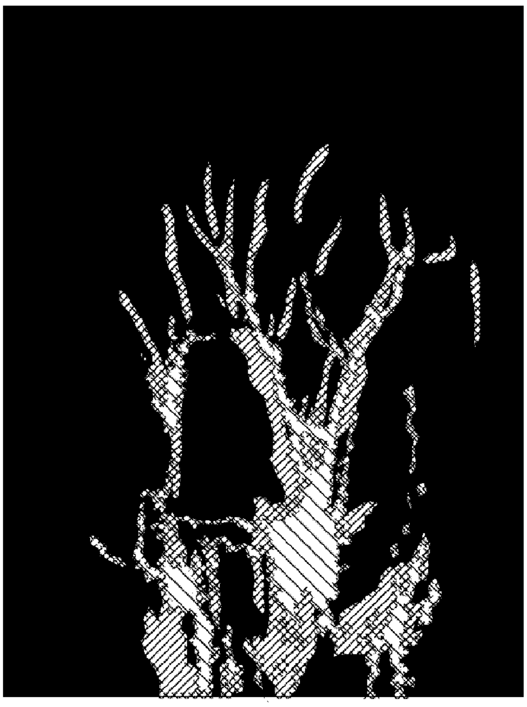

FIG.12

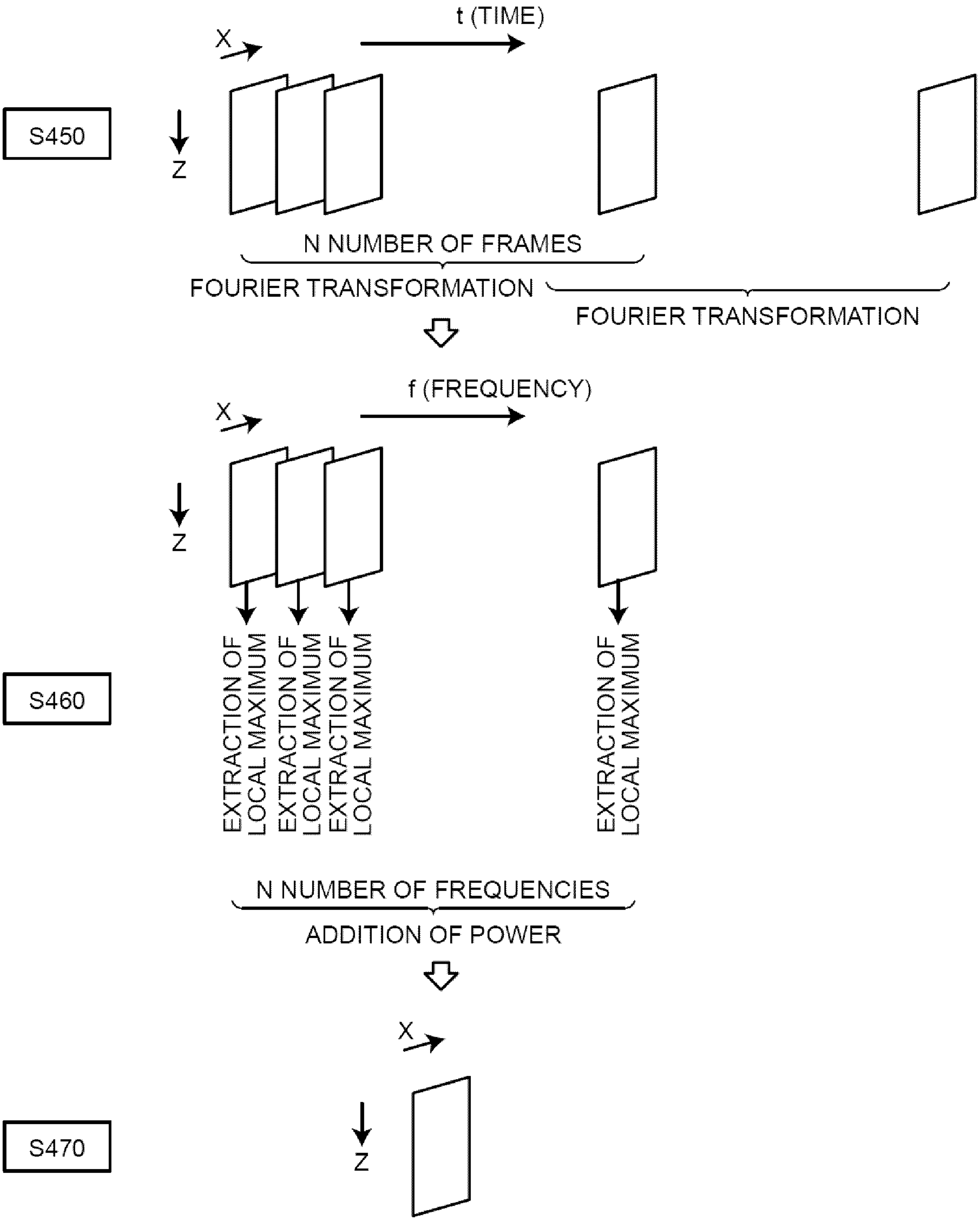
S450
t (TIME)
X
Z
N NUMBER OF FRAMES
FOURIER TRANSFORMATION
FOURIER TRANSFORMATION
S460
f (FREQUENCY)
X
Z
EXTRACTION OF LOCAL MAXIMUM
EXTRACTION OF LOCAL MAXIMUM
EXTRACTION OF LOCAL MAXIMUM
EXTRACTION OF LOCAL MAXIMUM
N NUMBER OF FREQUENCIES
ADDITION OF POWER
S470
X
Z

European Patent Office

# EUROPEAN SEARCH REPORT

Application Number
EP 24 20 7437

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | EP 1 128 768 B1 (SPENCER TECHNOLOGIES INC [US]) 7 January 2009 (2009-01-07)<br>* abstract *<br>* figures 1-16 *<br>* paragraph [0002] - paragraph [0048] *<br>----- | 1-6<br>7-14 | INV.<br>A61B8/00<br>A61B8/06<br>A61B8/08 |
| X | MEOLA MARIO ET AL: "Basics for performing a high-quality color Doppler sonography of the vascular access",<br>THE JOURNAL OF VASCULAR ACCESS, [Online]<br>vol. 22, no. 1_suppl,<br>2 November 2021 (2021-11-02), pages 18-31, XP093236281,<br>IT<br>ISSN: 1129-7298, DOI: 10.1177/11297298211018060<br>Retrieved from the Internet:<br>URL:https://pmc.ncbi.nlm.nih.gov/articles/PMC8607315/pdf/10.1177_11297298211018060.pdf><br>[retrieved on 2024-12-20]<br>* abstract *<br>* figures 1-13 *<br>* page 18 - page 30 *<br>----- | 6 | TECHNICAL FIELDS SEARCHED (IPC)<br>A61B |
| Y | ZHANG NAIYUAN ET AL: "Clutter suppression in ultrasound: performance evaluation and review of low-rank and sparse matrix decomposition methods",<br>BIOMEDICAL ENGINEERING ONLINE,<br>vol. 19, no. 1,<br>2 December 2020 (2020-12-02), XP093236279,<br>GB<br>ISSN: 1475-925X, DOI: 10.1186/s12938-020-00778-z<br>* abstract *<br>* figures 1-12 *<br>* page 1 - page 34 *<br>----- | 7-14 | |

The present search report has been drawn up for all claims

2

| Place of search | Date of completion of the search | Examiner |
|---|---|---|
| Munich | 7 January 2025 | Moehrs, Sascha |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 20 7437

This annex lists the patent family members relating to the patent documents cited in the above-mentioned European search report. The members are as contained in the European Patent Office EDP file on
The European Patent Office is in no way liable for these particulars which are merely given for the purpose of information.

07-01-2025

| Patent document cited in search report | Publication date | Patent family member(s) | Publication date |
|---|---|---|---|
| EP 1128768 B1 | 07-01-2009 | AT E419791 T1 | 15-01-2009 |
| | | AU 765304 B2 | 11-09-2003 |
| | | CA 2350669 A1 | 18-05-2000 |
| | | EP 1128768 A1 | 05-09-2001 |
| | | JP 2002529134 A | 10-09-2002 |
| | | US 6196972 B1 | 06-03-2001 |
| | | US 6616611 B1 | 09-09-2003 |
| | | US 2005075568 A1 | 07-04-2005 |
| | | WO 0027288 A1 | 18-05-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **JORGEN ARENDT JENSEN** ; **MIKKEL SCHOU** ; **SOFIE BECH ANDERSEN et al.** Fast Super Resolution Ultrasound Imaging using the Erythrocytes. *Proceedings Volume 12038, Medical Imaging 2022: Ultrasonic Imaging and Tomography*, 2022, vol. 12038, 120380E